# FASCICULE DE BREVET EUROPEEN

(11) **EP 0 507 691 B1**
(45) Date de publication et mention de la délivrance du brevet: **15.03.1995**
(21) Numéro de dépôt: 92400932.7
(22) Date de dépôt: 03.04.1992
(51) Int. Cl.: C07D 251/50, C07D 251/52, C07D 251/54, C07D 251/68, C07D 251/70, A61K 7/42

(54) **Dérivés s-triaziniques portant des substituants benzalmalonates, leur procédé de préparation, compositions cosmétiques filtrantes les contenant et leur utilisation pour protéger la peau et les cheveux du rayonnement ultraviolet**
S-Triazinderivate mit Benzalmalonaten als Substituenten, Verfahren zu ihrer Herstellung, diese enthaltende kosmetische Zubereitungen mit filternder Wirkung und ihre Verwendung als Schutzmittel für Haut und Haar
S-triazine derivatives substituted with benzalmalonates, process for their preparation, filtering cosmetic compositions containing them and their use as UV-protectors for skin and hair

(30) Priorité: 04.04.1991 FR 9104122
(43) Date de publication de la demande: 07.10.1992
(73) Titulaire: L'OREAL, 75008 Paris (FR)
(72) Inventeur: Richard, Hervé, F-93240 Villepinte (FR); Leduc, Madeleine, F-75011 Paris (FR); Junino, Alex, F-92180 Livry-Gargan (FR)
(74) Mandataire: Casalonga, Axel

(56) Documents cités:
- EP-A- 0 087 098
- GB-A- 935 515
- SEIFEN,OLE,FETTE,WACHSE vol. 115, no. 18, 21 Novembre 1989, AUGSBURG,DE pages 661 - 662; K.SPERLING: 'UV-Filter für Haut-und Produktschutz in kosmetischen Formulierungen'

## Description

La présente invention concerne de nouveaux dérivés s-triaziniques portant des substituants benzalmalonates, leur procédé de préparation et leur utilisation en tant que filtres solaires, notamment dans le domaine cosmétique.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage; ce rayonnement UV-B doit donc être filtré.

On sait également que les rayons UV-A de longueurs d'onde comprises entre 320 et 400 nm, provoquant le brunissement de la peau, sont susceptibles d'induire une altération de celle-ci notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques.

Il est donc intéressant de disposer de composés susceptibles d'absorber aussi bien les rayons UV-A que les rayons UV-B nocifs pour la peau.

Il est également souhaitable d'assurer aux cheveux une bonne protection contre la dégradation photochimique afin d'éviter en particulier un changement de nuance ou une décoloration.

On sait par ailleurs que les constituants entrant dans les préparations cosmétiques ne possèdent pas toujours une stabilité suffisante à la lumière et se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement utilisés en cosmétique et, en particulier, dans les huiles et graisses.

On connaît, par le brevet US 4 617 390, des dérivés de s-triazine portant des substituants p-aminobenzoates, qui n'absorbent que le rayonnement UV-B et dont la solubilité dans les corps gras est limitée; il est donc nécessaire, si l'on souhaite absorber l'ensemble du rayonnement UV, d'associer à ces dérivés de s-triazine greffés par des restes p-aminobenzoates, des filtres UV-A.

La demanderesse a découvert de nouveaux dérivés s-triaziniques greffés par des restes pouvant absorber le rayonnement UV-A ou l'ensemble du rayonnement UV (UV-B et UV-A) et possédant en outre une excellente solubilité dans les corps gras. Ces dérivés s-triaziniques possèdent également un coefficient d'absorption molaire très élevé, ce qui permet de les utiliser à faible concentration, par exemple dans les compositions cosmétiques de protection de la peau ou anti-solaires. Associés à des filtres UV-A du type dibenzoylméthane, notamment le 4-tert.-butyl-4'-méthoxydibenzoylméthane (Parsol 1789), ils permettent de photostabiliser ces composés.

Outre leurs propriétés filtrantes et leur excellent caractère liposoluble, ces nouveaux dérivés s-triaziniques présentent une bonne stabilité chimique et photochimique et ont l'avantage de n'être ni toxiques, ni irritants et d'avoir une parfaite innocuité vis-à-vis de la peau.

La présente invention a donc pour objet les nouveaux composés répondant à la formule suivante (I) : 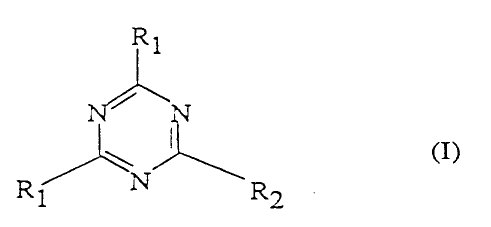
dans laquelle R₁ répond à la formule : 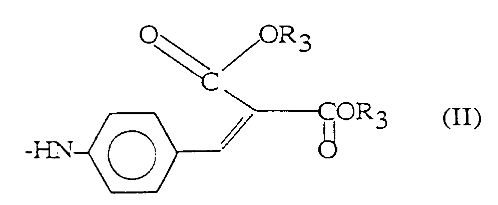
où R₃ représente un radical alkyle linéaire ou ramifié contenant de 1 à 20 atomes de carbone;
R₂ est identique à R₁, ou est un halogène, un groupement alcoxy ou mono- ou di-alkylamino linéaire ou ramifié contenant de 1 à 20 atomes de carbone ou un groupement répondant aux formules (III) ou (IV) ci-après : 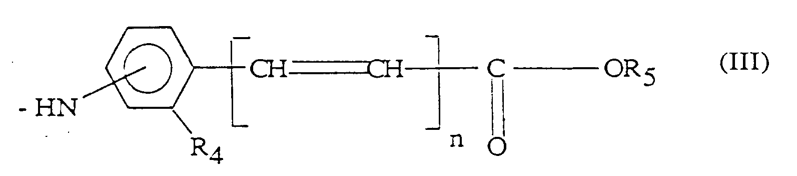
dans laquelle R₅ est un radical alkyle linéaire ou ramifié contenant de 1 à 20 atomes de carbone, et n = 0 ou 1;
- lorsque n est égal à 0, R₄ représente un atome d'hydrogène, le reste amino se trouvant en position 4 par rapport au groupement carboxyle, ou R₄ représente un radical hydroxyle ou un radical alcoxy en C₁-C₆, le reste amino se trouvant en position 4 ou 5 par rapport au groupement carboxyle;
- lorsque n est égal à 1, R₄ représente un atome d'hydrogène, le reste amino se trouvant en position 4 par rapport au groupement insaturé, 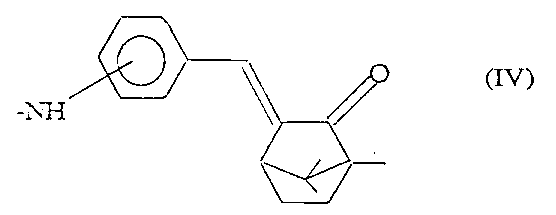 dans laquelle le reste amino se trouve en position 2, 3 ou 4 par rapport au groupement méthylidène camphre,
   sous réserve que :
- lorsque R₂ = R₁, R₃ est un radical comportant au moins 4 atomes de carbone;
- lorsque R₂ ≠ R₁ et répond à la formule (III), R₅ comporte au moins 4 atomes de carbone lorsque R₃ comporte de 1 à 7 atomes de carbone;
- lorsque R₂ ≠ R₁ et R₂ ne répond pas à la formule (III), mais est un radical alcoxy ou mono- ou di-alkylamino, R₂ comporte au moins 6 atomes de carbone lorsque R₃ comporte de 1 à 3 atomes de carbone.

Parmi les radicaux alkyle linéaires ou ramifiés, on peut citer par exemple : méthyle, éthyle, n-propyle, isopropyle, n-butyle, isobutyle, tert.-butyle, n-amyle, isoamyle, néopentyle, n-hexyle, n-heptyle, n-octyle, 2-éthylhexyle, n- dodécyle, n-octadécyle.

Parmi les radicaux alcoxy on peut citer méthoxy, éthoxy, n-propoxy, isopropoxy, n-butoxy, isobutoxy, tert.-butoxy, n-amyloxy, isoamyloxy, néopentyloxy, n-hexyloxy, n-heptyloxy, n-octyloxy, 2-éthylhexyloxy, n-dodécyloxy, n-octadécyloxy.

Parmi les halogènes, on peut citer plus particulièrement le chlore et le brome.

Ces nouveaux composés absorbent le rayonnement UV-A lorsque R₂ est identique à R₁ ou est un halogène, un groupement alcoxy ou mono- ou di-alkylamino ou répond à la formule (IV) ou (III) dans laquelle n est égal à 1, ou n est égal à 0, et R₄ représente un radical hydroxyle, le reste amino se trouvant en position 5 par rapport au groupement carboxyle, ou absorbent le rayonnement UV large bande lorsque R₂ répond à la formule (III) dans laquelle n est égal à 0, et R₄ représente un atome d'hydrogène ou un radical hydroxyle, le reste amino se trouvant en position 4 par rapport au groupement carboxyle, ou un radical alcoxy en C₁-C₆, le reste amino se trouvant en position 4 ou 5 par rapport au groupement carboxyle.

Ces nouveaux dérivés de s-triazines peuvent être utilisés comme filtres solaires pour la peau humaine et les cheveux ainsi que comme agents protecteurs de la lumière dans l'industrie des plastiques.

La présente invention a également pour objet le procédé de préparation des composés de formule (I).

Les composés de formule (I) peuvent être obtenus selon le schéma réactionnel ci-dessous : 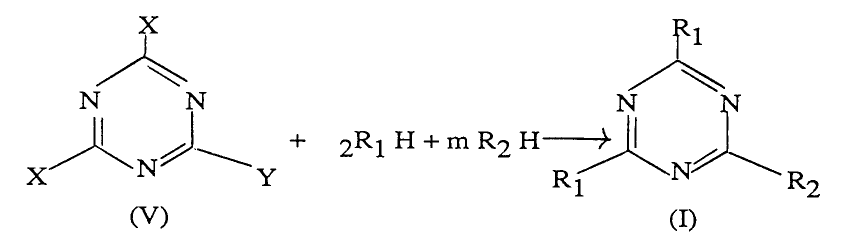
où R₁ répond à la formule (II) indiquée ci-dessus et R₂ a la même signification que ci-dessus; X représente un halogène, en particulier le chlore ou le brome, Y représente un halogène, en particulier le chlore ou le brome, ou un groupement alcoxy ou mono- ou di-alkylamino linéaire ou ramifié contenant de 1 à 20 atomes de carbone; m est un nombre entier égal à 1 si R₂ répond à la formule (II), (III) ou (IV); dans les autres cas, m est égal à 0.

Pour obtenir le composé (I) dans lequel R₂ est identique à R₁ et a la formule (II), on doit utiliser un excès stoechiométrique de composé R₁H de l'ordre de 10% à 50% par rapport au composé (V) dans lequel Y est un halogène.

Dans le cas contraire, on obtient un mélange de composé (I) dans lequel R₂ est identique à R₁ et a la formule (II) et de composé (I) dans lequel R₂ est un halogène. Ce mélange peut être séparé par des moyens classiques tels que chromatographie, cristallisation fractionnée, etc.

Les composés de formule générale (V) dans lesquels Y est différent de X peuvent être obtenus selon le schéma réactionnel ci-dessous, qui est décrit dans les publications : J.T. THURSTON et al, J. Am. Chem. Soc., 73, 2981 (1951) et J.R. DUDLEY et al., J. Am. Chem. Soc. 73, 2986 (1951) : 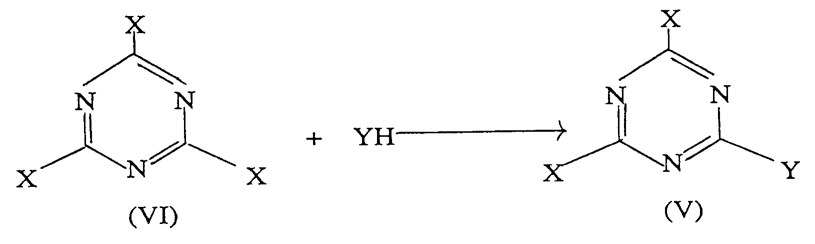
X et Y ayant les mêmes significations que ci-dessus.

Les réactions ci-dessus peuvent être effectuées éventuellement en présence d'un solvant (par exemple : toluène, xylène ou acétone/eau), à une température comprise entre 0 et 250°C, plus particulièrement entre 0 et 150°C.

Les composés R₁H et R₂H où R₂ a la formule (II) ou (III) peuvent être préparés selon des méthodes connues décrites dans les brevets FR-2 151 503, FR-A 2 385 685, GB 1 064 116.

Les composés R₂H où R₂ à la formule (IV) c'est-à-dire les ortho, méta et para-aminobenzylidène camphres, peuvent être préparés de la manière suivante à partir des dérivés nitro benzylidène camphres correspondants.

### Synthèse des ortho et para nitro benzylidène camphres

A 10°C, on nitre du D,L-benzylidène camphre (200 g, 0.83 mole) en solution dans 380 ml d'acide sulfurique à 98%, par addition goutte à goutte d'un mélange de 84,2 g d'acide nitrique à 65% et de 45 ml d'acide sulfurique à 98%. On maintient l'agitation 1 heure à 10-20°C après la fin de l'introduction, puis on verse le mélange réactionnel dans 4 litres de glace sous forte agitation. Le précipité est lavé à l'eau puis recristallisé deux fois dans l'isopropanol. On obtient 115 g (rendement = 48%) de 4-nitro benzylidène camphre :
- poudre blanc cassé
- Pf = 158°C
- UV : (éthanol à 950) λₘₐₓ = 314 nm (εₘₐₓ = 22800)
- Spectre de RMN en accord avec la formule.

Par concentration des jus de recristallisation à demi-filtration du précipité et nouvelles recristallisations dans l'éthanol puis dans l'éther diisopropylique, on isole l'ortho nitro benzylidène camphre :
- Poudre jaune pâle
- Pf = 118°C
- UV : (éthanol à 95°) λₘₐₓ = 256 nm (εₘₐₓ = 16350)
   λₘₐₓ = 320 nm (épaulement) (εₘₐₓ = 4390)
- Spectres de RMN en accord avec la formule.

### Synthèse du para amino benzylidène camphre

On porte au reflux un mélange de zinc en poudre fine (60 g), d'éthanol (250 ml), de chlorure d'ammonium (2 g) et d'eau (20 ml) et on ajoute par portions, sous agitation, le para nitro benzylidène camphre (20 g, 0,07 mole). On laisse une demi-heure au reflux, on filtre le zinc et on le rince à l'alcool. Le filtrat est versé sur de la glace (500 g). Le précipité obtenu est filtré, lavé à l'eau et séché sous vide. On obtient un rendement quantitatif de para amino benzylidène camphre (17,7 g). Il est recristallisé dans l'éthanol pour donner le dérivé attendu :
- Poudre jaune pâle
- Pf = 144-145°C
- UV : (éthanol à 95°) λₘₐₓ = 357 nm (εₘₐₓ = 27500)
- Analyse : C₁₇H₂₁NO

| | C% | H% | N% | O% |
|---|---|---|---|---|
| Calculé | 79,96 | 8,29 | 5,49 | 6,27 |
| Trouvé | 79,94 | 8,31 | 5,53 | 6,42 |

### Synthèse de l'ortho amino benzylidène camphre

De la même manière que ci-dessus, en partant de l'ortho nitro benzylidène camphre, on obtient le dérivé ortho amino benzylidène camphre :
- Poudre jaune citron
- Pf = 155°C
- UV : (éthanol à 95°) λₘₐₓ = 290 nm (εₘₐₓ = 9880)
   λₘₐₓ = 366 nm (εₘₐₓ = 5870)
- Spectres de RMN en accord avec la formule.

### Synthèse du méta nitro benzylidène camphre

Dans un ballon, on place le camphre (50,2 g, 0,33 mole), du diméthoxy éthane (180 ml) et de l'hydrure de sodium (32 g à 50% dans l'huile, 0,33 mole) lavé avec du diméthoxy éthane. On chauffe sous azote à 80°C et on ajoute le méta nitro benzaldéhyde (45,3 g 0,3 mole) dissous dans du diméthoxy éthane (100 ml) goutte à goutte en une demi-heure. On laisse sous agitation à 80°C pendant 1,5 heure. On laisse refroidir le mélange réactionnel et on le verse prudemment dans 2 litres d'eau acidifiée et glacée. Le précipité brun obtenu est filtré, lavé à l'eau et séché. Après recristallisation dans l'éther diisopropylique en présence de noir végétal, on obtient 20 g de méta nitro benzylidène camphre.
- Poudre beige clair
- Pf = 113°C
- Spectre de RMN en accord avec la formule.

### Synthèse du méta amino benzylidène camphre

La réduction du méta nitro benzylidène camphre est conduite de la même façon que celle des ortho et para nitro benzylidène camphres précédents. On obtient, après recristallisation dans l'éther diisopropylique, le méta amino benzylidène camphre :
- Poudre jaune pâle
- Pf = 77-78°C
- UV : (éthanol à 95°) λₘₐₓ = 295 nm (εₘₐₓ = 18950)
- Analyse : C₁₇H₂₁NO

| | C% | H% | N% | O% |
|---|---|---|---|---|
| Calculé | 79,96 | 8,29 | 5,49 | 6,27 |
| Trouvé | 79,81 | 8,36 | 5,49 | 6,48 |

Le p-aminobenzylidène camphre, qui est un composé connu, peut aussi être préparé comme décrit dans HALLER, BOUDIN, Annales de Chimie, 9e série, tome XVII (1922).

Parmi les composés de formule (I) ci-dessus, on peut citer plus particulièrement :
1) . 2,4,6-tris(4'-amino benzalmalonate de diisobutyle)-s-triazine
2) . 2,4-bis[4'-amino benzalmalonate de di(2-éthylhexyle)]-6-chloro-s-triazine
3) . 2,4,6-tris[4'-amino benzalmalonate de di(2-éthylhexyle)]-s-triazine
4) . 2,4-bis[(4'-amino benzalmalonate de di(2-éthylhexyle)]-6-(4'-amino benzoate de 2-éthylhexyle)-s-triazine.
5) . 2,4-bis(4'-amino benzalmalonate de diisobutyle)-6-butoxy-s-triazine
6) . 2,4-bis(4'-amino benzalmalonate de diisobutyle)-6-(2-éthylhexylamino)-s-triazine.
7) . 2,4-bis (4'-amino benzalmalonate de diisobutyle)-6-(5'-amino salicylate de 2-éthylhexyle)-s-triazine
8) . 2,4-bis(4'-amino benzalmalonate de diisobutyle)-6-(4'-amino cinnamate de 2-éthylhexyle)-s-triazine
9) . 2,4-bis(4'-aminobenzalmalonate de diisobutyle)-6-(4'aminobenzylidène camphre)-s-triazine.

De par leur caractère liposoluble, les composés de formule (I) ci-dessus se répartissent uniformément dans les supports cosmétiques classiques contenant au moins une phase grasse ou un solvant organique cosmétiquement acceptable et peuvent être appliqués sur la peau ou les cheveux pour constituer un film protecteur efficace.

La présente invention a donc aussi pour objet une composition cosmétique comprenant, dans un support cosmétiquement acceptable contenant au moins une phase grasse ou un solvant organique, une quantité efficace d'au moins un composé de formule (I) ci-dessus.

La composition cosmétique de l'invention peut être utilisée comme composition protectrice de l'épiderme humain ou des cheveux ou comme composition anti-solaire.

La présente invention a également pour objet un procédé de protection de la peau et des cheveux naturels ou sensibilisés vis-à-vis du rayonnement solaire, consistant à appliquer sur la peau ou les cheveux une quantité efficace d'une composition cosmétique contenant au moins un composé de formule (I).

On entend par "cheveux sensibilisés" des cheveux ayant subi un traitement de permanente, de coloration ou de décoloration.

L'invention a également pour objet une composition cosmétique colorée ou non colorée, stabilisée à la lumière, comprenant une quantité efficace d'au moins un composé de formule (I) ci-dessus.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotions huileuses ou oléoalcooliques, d'émulsions telles qu'une crème ou un lait, de dispersions vésiculaires de lipides amphiphiles ioniques ou non-ioniques, de gels oléoalcooliques ou alcooliques, de bâtonnets solides ou être conditionnée en aérosol. Les crèmes constituent ce que l'on appelle généralement les "crèmes de jour protectrices" d'usage quotidien.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Elle peut contenir en outre d'autres filtres UV-A et/ou UV-B.

Le composé de formule (I) est présent dans des proportions en poids comprises entre 0,1 et 2% par rapport au poids total de la composition cosmétique protectrice de l'épiderme humain.

Comme solvant de solubilisation, on peut utiliser une huile, une cire et de façon générale tout corps gras, un monoalcool ou un polyol inférieur ou leurs mélanges. Les monoalcools ou polyols plus particulièrement préférés sont l'éthanol, l'isopropanol, le propylèneglycol, l'hexylèneglycol, la glycérine et le sorbitol.

Une forme de réalisation de l'invention est une émulsion sous forme de crème ou de lait protecteurs comprenant en plus du composé de formule (I), des alcools gras, des esters d'acides gras et notamment des triglycérides d'acides gras, des acides gras, de la lanoline, des huiles ou cires naturelles ou synthétiques et des émulsionnants, en présence d'eau.

Les dispersions vésiculaires de lipides amphiphiles ioniques ou non-ioniques peuvent être préparées selon des procédés connus.

On peut, par exemple, faire gonfler les lipides dans une solution aqueuse pour former des sphérules dispersées dans le milieu aqueux comme décrit dans l'article BANGHAN, STANDISH & WATKINS, J. Mol. Biol., 13,238 (1965) ou dans les brevets FR-2 315 991 et 2 416 008 de la demanderesse.

Une autre forme de réalisation est constituée par des lotions huileuses à base d'huiles et cires naturelles ou synthétiques, de lanoline, et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylèneglycol ou la glycérine et un épaississant tel que la silice. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

Dans le cas d'une composition conditionnée en aérosol, on utilise les propulseurs classiques.

La présente invention vise également les compositions cosmétiques anti-solaires contenant au moins un composé de formule (I) et pouvant contenir d'autres filtres UV-B et/ou UV-A.

Dans ce cas, la quantité totale de filtres présents dans la composition anti-solaire, c'est-à-dire le composé de formule (I) et éventuellement les autres filtres, est comprise entre 0,3 et 15% en poids par rapport au poids total de la composition anti-solaire.

Ces compositions anti-solaires se présentent sous les formes indiquées ci-dessus pour les compositions protectrices de l'épiderme humain.

A titre de filtres solaires filtrant les rayons UV-B, on peut citer les filtres hydrosolubles tels que les dérivés du benzylidène camphre décrits dans les brevets français 2 199 971, 2 236 515, 2 282 426 et 2 383 904 de la demanderesse et plus particulièrement le méthylsulfate de 4-(2-oxo-3-bornylidène-méthyl)phényltriméthyl ammonium, les sels de l'acide 4-(2-oxo-3-bornylidène méthyl)benzène sulfonique, de l'acide 2-méthyl-5-(2-oxo-3-bornylidèneméthyl)benzène sulfonique, de l'acide 3-benzylidène-2-oxo-10-bornanesulfonique et de l'acide 2-phénylbenzimidazole-5-sulfonique.

Les composés selon l'invention peuvent également être associés à des filtres UV-B constitués par des composés liposolubles ou par des huiles ayant des propriétés filtrantes telles qu'en particulier l'huile de café. A titre de filtres solaires UV-B lipophiles, on peut citer les dérivés de l'acide salicylique tels que le salicylate de 2-éthylhexyle, le salicylate d'homomenthyle, les dérivés de l'acide cinnamique tels que le p-méthoxycinnamate de 2-éthylhexyle, le p-méthoxycinnamate de 2-éthoxyéthyle, les dérivés de l'acide p-aminobenzoïque tels que le p-aminobenzoate d'amyle, le p-diméthylaminobenzoate de 2-éthyl hexyle, les dérivés de benzophénone tels que la 2-hydroxy 4-méthoxybenzophénone, la 2,2'-dihydroxy 4-méthoxybenzophénone, les dérivés du camphre tels que le 3-(4'-méthylbenzylidène)camphre ou le 3-benzylidènecamphre.

Les composés selon l'invention peuvent aussi être associés à des filtres UV-A parmi lesquels on peut citer les dérivés du dibenzoylméthane, par exemple le 2-méthyl dibenzoylméthane, le 4-méthyldibenzoylméthane, le 4-isopropyldibenzoylméthane, le 4-tert.-butyl-dibenzoylméthane, le 2,4-diméthyldibenzoylméthane, le 2,5-diméthyldibenzoylméthane, le 4,4'-diisopropyldibenzoylméthane, le 4-tert.butyl-4'-méthoxydibenzoylméthane, le 2-méthyl-5-isopropyl-4'-méthoxydibenzoylméthane, le 2-méthyl-5-tert.-butyl-4'-méthoxydibenzoylméthane, le 2,4-diméthyl-4'-méthoxydibenzoylméthane et le 2,6-diméthyl-4-tert.-butyl-4'-méthoxydibenzoylméthane, ainsi que les dérivés du benzène 1,4-[di(3-méthylidène camphre)] sulfonés sur le radical méthyle en position 10 du camphre tels que décrits dans les brevets français 2 528 420 et 2 639 347.

Il est entendu que la liste de filtres solaires utilisés en association avec les composés (I) selon l'invention qui est indiquée ci-dessus n'est pas limitative.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de laque pour cheveux, de composition de permanente, de coloration ou décoloration des cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousse, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,1 à 2% en poids de composé de formule (I).

La présente invention vise également les compositions cosmétiques contenant au moins un composé de formule (I) à titre d'agent de protection contre les rayons ultraviolets constituées par des compositions capillaires telles que les laques pour cheveux, les lotions de mise en plis éventuellement traitantes ou démêlantes, les shampooings colorants, les compositions tinctoriales pour cheveux; par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière au cours du stockage.

De telles compositions contiennent 0,1 à 2% en poids de composé de formule (I).

Les composés (I) selon l'invention peuvent également être incorporés dans différentes matières organiques, et notamment les matières plastiques, en vue de les protéger contre le rayonnement ultraviolet.

L'invention sera mieux illustrée, sans toutefois être limitée, par les exemples de réalisation suivants.

### EXEMPLE 1 2,4,6-tris(4'-amino benzalmalonate de diisobutyle)-s-triazine

On chauffe au reflux pendant 10 heures, sous azote, le chlorure de cyanuryle (5,28 g 0,0286 mole) et le 4-amino benzalmalonate de diisobutyle (33,5 g, 0,105 mole) dans du xylène (135 ml). On concentre sous vide, on passe le mélange réactionnel sur colonne de silice (éluant : CH₂Cl₂) et on recristallise dans le méthanol pour obtenir le dérivé de l'exemple 1 (20,6 g; rendement = 68%):
- poudre jaune pâle
- Pf = 97°C
- UV : (éthanol à 95°) λₘₐₓ = 355 nm (εₘₐₓ = 119600)
- Analyse : C₅₇H₇₂N₆O₁₂

| | C% | H% | N% | O% |
|---|---|---|---|---|
| Calculé | 66,26 | 7,02 | 8,13 | 18,58 |
| Trouvé | 66,17 | 7,04 | 8,10 | 18,47 |

### EXEMPLES 2 et 3 2,4-bis[4'-amino benzalmalonate de di(2-éthyl-hexyle)]-6-chloro-s-triazine et 2,4,6-tris[4'-amino benzalmalonate de di(2-éthylhexyle)]-s-triazine

On chauffe au reflux pendant 10 heures sous azote, le chlorure de cyanuryle (4,88 g, 0,027 mole) et le 4-amino benzalmalonate de di(2-éthylhexyle) (35,2 g, 0,082 mole) dans du xylène (130 ml). On refroidit et on évapore le solvant sous vide. L'huile obtenue est chromatographiée sur colonne de silice (éluant : CH₂Cl₂). Dans les premières fractions, on obtient 2,2 g du dérivé de l'exemple 2 ayant pour caractéristiques :
- poudre jaune pâle
- Pf = 70°C
- UV : (éthanol à 95°) λₘₐₓ = 345 nm (εₘₐₓ = 58100)
λₘₐₓ = 326 nm (εₘₐₓ = 57700)
- Analyse : C₅₅H₈₀ClN₅O₈

| | C% | H% | Cl% | N% | O% |
|---|---|---|---|---|---|
| Calculé | 67,77 | 8,27 | 3,64 | 7,18 | 13,13 |
| Trouvé | 67,80 | 8,30 | 3,76 | 7,20 | 13,34 |

Dans les fractions suivantes, on obtient 20,8 g du dérivé de l'exemple 3 ayant pour caractéristiques :
- cire jaune
- UV = (éthanol à 95°) λₘₐₓ = 355 nm (εₘₐₓ = 108000)
- Analyse : C₈₁H₁₂₀N₆O₁₂

| | C% | H% | N% | O% |
|---|---|---|---|---|
| Calculé | 71,02 | 8,83 | 6,13 | 14,02 |
| Trouvé | 71,18 | 8,81 | 6,21 | 13,88 |

### EXEMPLE 4 2,4-bis[4'-amino benzalmalonate de di(2-éthylhexyle)]-6-(4'-aminobenzoate de 2-éthylhexyle)-s-triazine

Le dérivé de l'exemple 2 (0,97 g, 10⁻³ mode) est porté au reflux dans le xylène (1,6 ml) sous azote pendant 6 heures avec du 4-amino benzoate de 2-éthylhexyle (0,25 g, 10⁻³ mole). Après refroidissement, le solvant est évaporé sous vide et l'huile obtenue est chromatographiée sur silice pour donner 0,95 g du dérivé de l'exemple 4 ayant pour caractéristiques :
- cire jaune
- UV : (éthanol à 95°) λₘₐₓ = 340 nm (εₘₐₓ = 79400)
λₘₐₓ = 300 nm (épaulement)(εₘₐₓ = 43300
- Analyse : C₇₀H₁₀₂N₆O₁₀

| | C% | H% | N% | O% |
|---|---|---|---|---|
| Calculé | 70,79 | 8,66 | 7,08 | 13,47 |
| Trouvé | 70,83 | 8,72 | 7,17 | 13,71 |

### EXEMPLE 5 2,4-bis(4'-amino benzalmalonate de diisobutyle)-6-butoxy-s-triazine

On chauffe au reflux pendant 3 heures la 2-butoxy-4,6-dichloro-s-triazine (11,1 g, 0,05 mole) avec du 4-amino benzalmalonate de diisobutyle (35,13 g, 0,11 mode) dans du toluène (350 ml). Après refroidissement, on ajoute une solution de bicarbonate de sodium saturée pour neutraliser et la phase organique est lavée à l'eau et séchée sur sulfate de sodium. Après élimination du solvant, le résidu obtenu est chromatographié sur colonne de silice (éluant : CH₂Cl₂) pour donner 4 g du dérivé de l'exemple 5 ayant pour caractéristiques :
- poudre à reflets jaunes
- Pf = 104-110°C
- UV : (éthanol à 95°) λₘₐₓ = 348 nm (εₘₐₓ = 62000)
- Analyse : C₄₃H₅₇N₅O₉

| | C% | H% | N% | O% |
|---|---|---|---|---|
| Calculé | 65,55 | 7,29 | 8,89 | 18,27 |
| Trouvé | 65,15 | 7,27 | 8,79 | 18,56 |

### EXEMPLE 6 2,4-bis(4'-amino benzalmalonate de diisobutyle)-6-(2-éthylhexylamino)-s-triazine

Dans un ballon, on introduit goutte à goutte une solution de chlorure de cyanuryle (0,2 mode, 36,8 g) dans l'acétone (300 ml) à de la glace pilée (100 g) sous vive agitation, puis une solution de 2-éthylhexylamine dans l'acétone (100 ml) en maintenant la température entre 0 et 5°C, enfin une solution de carbonate de sodium (11,2 g, 0,1 mole) dans 300 ml d'eau. On laisse une heure sous agitation à 0-5°C. On extrait l'huile au dichlorométhane, on lave la phase organique à l'eau et on sèche sur sulfate de sodium. Après évaporation du solvant, on obtient la 2-(2-éthylhexylamino)-4,6-dichloro-s-triazine (52 g, rendement = 95%) ayant pour caractéristiques :
- poudre blanche
- Pf = 37°C
- Analyse : C₁₁H₁₈Cl₂N₄

| | C% | H% | Cl% | N% |
|---|---|---|---|---|
| Calculé | 47,66 | 6,55 | 25,58 | 20,21 |
| Trouvé | 47,89 | 6,60 | 25,79 | 20,16 |

Le dérivé précédent (13,8 g, 0,05 mole) est porté au reflux dans le xylène (230 ml) sous azote pendant 8 heures avec du 4-amino benzal malonate de diisobutyle (35,13 g, 0,11 mole). Après refroidissement, on verse le mélange dans de l'eau glacée et on neutralise avec une solution saturée de carbonate de sodium. On extrait au dichlorométhane et on lave deux fois la phase organique avec de l'eau. On obtient une gomme marron qui est chromatographiée sur silice pour donner 23 g du dérivé de l'exemple 6 ayant pour caractéristiques :
- poudre jaune pâle amorphe
- UV : (éthanol à 95°) λₘₐₓ = 354 nm (εₘₐₓ = 70550)
- Analyse : C₄₇H₆₆N₆O₈

| | C% | H% | N% | O% |
|---|---|---|---|---|
| Calculé | 66,96 | 7,89 | 9,97 | 15,18 |
| Trouvé | 66,86 | 7,87 | 9,94 | 15,20 |

### EXEMPLE 7 2,4-bis(4'-amino benzalmalonate de diisobutyle)-6-(5'-aminosalicylate de 2-éthylhexyle)-s-triazine

Dans un ballon, on place le chlorure de cyanuryle (0,05 mole, 9,22 g) dans l'acétone (50 ml). On refroidit à 5-10°C, on ajoute 50 ml d'eau puis, goutte à goutte, le 5-amino salicylate de 2-éthylhexyle (0,05 mole, 13,26 g) dans 50 ml d'acétone. On ajoute ensuite le bicarbonate de sodium (0,05 mole, 4,2 g) en solution dans 100 ml d'eau et on laisse deux heures sous agitation. Le précipité obtenu est essoré et recristallisé dans l'éthanol pour donner la 2-(5'-aminosalicylate de 2-éthylhexyle)-4,6-dichloro-s-triazine (17 g, rendement = 83%) ayant pour caractéristiques :
- poudre blanche
- Pf = 131°C
- Analyse : C₁₈H₂₂Cl₂N₄O₃

| | C% | H% | Cl% | N% | O% |
|---|---|---|---|---|---|
| Calculé | 52,31 | 5,37 | 17,16 | 13,56 | 11,61 |
| Trouvé | 52,50 | 5,39 | 17,08 | 13,48 | 11,83 |

Le dérivé précédent (4,13 g 0,01 mole) est porté au reflux dans le xylène (47 ml) sous azote pendant 10 heures avec du 4-amino benzalmalonate de diisobutyle (6,7 g, 0,021 mole). Après refroidissement, on verse le mélange dans de l'eau glacée et on neutralise avec une solution de bicarbonate de sodium. La phase organique est lavée deux fois à l'eau et séchée sur sulfate de sodium. Après élimination du solvant, l'huile obtenue est chromatographiée sur silice (éluant : CH₂Cl₂) pour donner le composé de l'exemple 7 ayant pour caractéristiques :
- poudre jaune pâle amorphe
- UV : (éthanol à 95°) λₘₐₓ = 355 nm (εₘₐₓ = 82120)
- Analyse : C₅₄H₇₀N₆O₁₁

| | C% | H% | N% | O% |
|---|---|---|---|---|
| Calculé | 66,24 | 7,21 | 8,58 | 17,97 |
| Trouvé | 66,25 | 7,25 | 8,39 | 17,89 |

### EXEMPLE 8 2,4-bis(4'-amino benzalmalonate de diisobutyle)-6-(4'-aminocinnamate de 2-éthylhexyle)-s-triazine

Dans un ballon, on place le chlorure de cyanuryle (0,01 mole, 1,85 g) dans l'acétone (10 ml). On refroidit à 5-10°C. On ajoute 5 ml d'eau puis, goutte à goutte, le 4-amino cinnamate de 2-éthylhexyle (0,01 mole, 2,75 g) dans 50 ml d'acétone. On ajoute ensuite le bicarbonate de sodium (0,01 mole, 0,84 g) en solution dans 50 ml d'eau et on laisse une demi-heure sous agitation. Le précipité obtenu est essoré et séché pour donner la 2-(4'-amino cinnamate de 2-éthylhexyle)-4,6-dichloro-s-triazine (4 g, rendement = 94%) ayant pour caractéristiques :
- poudre jaune pâle
- Pf = 140°C
- Analyse : C₂₀H₂₄Cl₂N₄O₂

| | C% | H% | Cl% | N% | O% |
|---|---|---|---|---|---|
| Calculé | 56,74 | 5,71 | 16,75 | 13,23 | 7,56 |
| Trouvé | 56,78 | 5,80 | 16,89 | 13,10 | 7,80 |

Le dérivé précédent (3,17 g, 7,5 10⁻³ mole) est porté au reflux dans le xylène (35 ml) sous azote pendant 7 heures avec du 4-amino benzal malonate de diisobutyle (5,27 g, 0,016 mole). Après refroidissement, on neutralise avec une solution saturée de carbonate de sodium. La phase organique est lavée deux fois à l'eau et séchée sur sulfate de sodium. Après élimination du solvant, l'huile obtenue est chromatographiée sur silice (éluant : CH₂Cl₂) pour donner le composé de l'exemple 8 (5,2 g, rendement = 70%) ayant pour caractéristiques :
- poudre jaune pâle amorphe
- UV : (éthanol à 95°) λₘₐₓ = 354 nm (εₘₐₓ = 113000)
- Analyse : C₅₆H₇₂N₆O₁₀

| | C% | H% | N% | O% |
|---|---|---|---|---|
| Calculé | 67,99 | 7,34 | 8,50 | 16,17 |
| Trouvé | 67,62 | 7,52 | 8,36 | 16,51 |

### EXEMPLE 9 2,4-bis(4'-amino benzalmalonate de diisobutyle)-6-(4'-amino benzylidène camphre)-s-triazine

Dans un ballon, on place le chlorure de cyanuryle (0,05 mole, 9,2g) dans l'acétone (100 ml). On refroidit à 5-10°C, on ajoute 50 ml d'eau puis, goutte à goutte, vers 0-5°C le 4-amino benzylidène camphre (0,05 mole, 12,7 g) dans 100 ml d'acétone. On ajoute ensuite le bicarbonate de sodium (0,05 mole, 4,2 g) en solution dans 50 ml d'eau et on laisse une demi-heure sous agitation. Le précipité obtenu est essoré et séché pour donner la 2-(4'-amino benzylidène camphre)-4,6-dichloro-s-triazine (19,5 g, rendement = 97%) ayant pour caractéristiques :
- poudre jaune pâle
- Pf = 250°C
- Analyse : C₂₀H₂₀Cl₂N₄O

| | C% | H% | Cl% | N% | O% |
|---|---|---|---|---|---|
| Calculé | 59,56 | 5,00 | 13,89 | 3,97 | 17,58 |
| Trouvé | 59,76 | 5,01 | 13,76 | 4,15 | 17,53 |

Le dérivé précédent (8,06 g, 0,02 mole) est porté au reflux dans le xylène (95 ml) sous azote pendant 6 heures avec du 4-amino benzal malonate de diisobutyle (14,05 g, 0,044 mole). Après refroidissement, on neutralise avec une solution saturée de bicarbonate de sodium. La phase organique est lavée deux fois à l'eau et séchée sur sulfate de sodium. Après élimination du solvant, la gomme obtenue est chromatographiée sur silice (éluant : CH₂Cl₂) pour donner le dérivé de l'exemple 9 (13,3 g, rendement = 69%) ayant pour caractéristiques :
- poudre jaune pâle amorphe
- UV : (éthanol à 95°) λₘₐₓ = 356 nm (εₘₐₓ = 108500)
- Analyse : C₅₆H₆₈N₆O₉

| | C% | H% | N% | O% |
|---|---|---|---|---|
| Calculé | 69,40 | 7,07 | 8,67 | 14,66 |
| Trouvé | 68,80 | 7,11 | 8,41 | 15,24 |

### EXEMPLES DE FORMULATION

### EXEMPLE A

### Crème Anti-solaire

| *Phase **A*** | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 7,0 g |
| - Mélange de mono- et distéarate de glycérol non autoémulsionnable | 2,0 g |
| - Alcool cétylique | 1,5 g |
| - Huile de silicone | 1,5 g |
| - Alcool myristique oxypropylèné à 3 moles d'oxyde de propylène vendu sous le nom de" WITCONOL APM" par la Société WITCO | 15,0 g |
| - 2,4 bis (4'-amino benzalmalonate de diisobutyle)-6-butoxy-s-triazine (exemple 5) | 3,0 g |
| - p-diméthylamino benzoate de 2-éthylhexyle | 4,0 g |

| *Phase **B*** | |
|---|---|
| - Glycérine | 20,0 g |
| - Eau qsp | 100,0 g |

| *Phase **C*** | |
|---|---|
| - Parfum, conservateurs des deux phases **A** et **B** qs | |

### MODE OPERATOIRE

- Les phases **A** et **B** sont chauffées au bain-marie à 80°C.
- La phase **A** est versée dans la phase **B** sous agitation vive pendant 5 minutes.
- On continue d'agiter jusqu'à 40°C.
- On ajoute ensuite le parfum et les conservateurs vers 40°C.
- On agite jusqu'à température ambiante.

### EXEMPLE B

### Crème Anti-solaire

| *Phase **A*** | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 7,0 g |
| - Mélange de mono- et distéarate de glycérol non autoémulsionnable | 2,0 g |
| - Alcool cétylique | 1,5 g |
| - Huile de silicone | 1,5 g |
| - Alcool myristique oxypropyléné à 3 moles d'oxyde de propylène vendu sous le nom de" WITCONOL APM" par la Société WITCO | 15,0 g |
| - 2,4,6-tris (4'-amino benzalmalonate de di(2-éthylhexyle)-s-triazine (exemple 3) | 5,0 g |

| *Phase **B*** | |
|---|---|
| - Sel de triéthanolamine de l'acide-2-phénylbenzimidazole-5-sulfonique | 3,0 g |
| - Glycérine | 20,0 g |
| - Eau qsp | 100,0 g |

| *Phase **C*** | |
|---|---|
| - Parfum, conservateurs des deux phases **A** et **B** qs | |

On procède de la même façon que dans l'exemple A.

### EXEMPLE C

### Crème protectrice de l'épiderme humain

| *Phase **A*** | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 7,0 g |
| - Mélange de mono- et distéarate de glycérol non autoémulsionnable | 2,0 g |
| - Alcool cétylique | 1,5 g |
| - Huile de silicone | 1,5 g |
| - Alcool myristique oxypropyléné à 3 moles d'oxyde de propylène vendu sous le nom de" WITCONOL APM" par la Société WITCO | 15,0 g |
| - 2,4-bis (4'-amino benzalmalonate de diisobutyle)-6-(4'-amino benzylidène camphre)-s-triazine (exemple 9) | 1,0 g |

| *Phase **B*** | |
|---|---|
| - Glycérine | 20,0 g |
| - Eau qsp | 100,0 g |

| *Phase **C*** | |
|---|---|
| - Parfum, conservateurs des deux phases **A** et **B** qs | |

On procède de la même façon que dans l'exemple A.

### EXEMPLE D

### Crème (huile-dans-l'eau)protectrice de l'épiderme humain

| | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 7,0 g |
| - Mélange de mono- et distéarate de glycérol non autoémulsionnable | 2,0 g |
| - Alcool cétylique | 1,5 g |
| - Huile de silicone | 1,5 g |
| - Triglycérides d'acides caprique/caprylique vendus sous la dénomination commerciale de" MIGLYOL 812" par la Société DYNAMIT NOBEL | 20,0 g |
| - 2,4,6-tris (4'-amino benzalmalonate de diisobutyle)-s-triazine (exemple 1) | 1,0 g |
| - 4-tert.-butyl-4'-méthoxy dibenzoylméthane vendu sous la dénomination commerciale de "PARSOL 1789" par la Société GIVAUDAN | 1,0 g |
| - Glycérine | 20,0 g |
| - Parfum, conservateurs qs | |
| - Eau qsp | 100,0 g |

On porte la phase huileuse contenant les émulsionnants et les filtres à 80°C.

On porte la phase aqueuse contenant la glycérine à la même température. On verse ensuite la phase huileuse dans la phase aqueuse sous vive agitation. On poursuit l'agitation en laissant refroidir. Vers 40°C, on ajoute parfum et conservateurs.

### EXEMPLE E

### Huile Anti-solaire

On mélange les ingrédients suivants en chauffant éventuellement à 40-45°C pour homogénéiser :

| | |
|---|---|
| - Huile de vaseline | 2,5 g |
| - 2,4,6-tris (4'-amino benzalmalonate de disobutyle)-- s-triazine (exemple 1) | 2,0 g |
| - ditert.-butyl hydroxy toluène | 0,05 g |
| - Parfum qs | |
| - Triglycérides d'acides gras en C₈-C₁₂ vendu sous la dénomination de "MIGLYOL 812" par la Société DYNAMIT NOBEL | 40 g |
| - p-méthoxycinnamate de 2-éthylhexyle | 2 g |
| - Alcool à 96° qsp | 100 g |

### EXEMPLE F

### Huile Anti-solaire

On mélange les ingrédients suivants en chauffant éventuellement à 40-45°C pour homogénéiser :

| | |
|---|---|
| - 2,4-bis [(4'-amino benzalmalonate de di(2-éthyl- hexyle)]-6-(4'-amino benzoate de 2-éthylhexyle)-s-triazine (exemple 4) | 3,0 g |
| - Alcool à 96° | 15 g |
| - Parfum qs | |
| - Propylène glycol | 10 g |
| - Adipate de diisopropyle vendu sous la dénomination commerciale de "CERAPHYL 230" par la Société Van DYK qsp | 100 g |

### EXEMPLE G

### Huile Anti-solaire

On mélange les ingrédients suivants en chauffant éventuellement à 40-45°C pour homogénéiser :

| | |
|---|---|
| - 2,4,-bis (4'-amino benzalmalonate de di 2-éthyl- hexyle)-6-(4'-amino benzoate de 2-éthylhexyle)-s-triazine (exemple 4) | 2,0 g |
| - Benzoate d'alcools gras en C₁₂-C₁₅ vendu sous la dénomination de "FINSOLV TN" par la Société FINETEX | 30 g |
| - Huile de tournesol | 20 g |
| - Parfum qs | |
| - Diméthyl polysiloxane cyclique vendu sous la dénomination "VOLATILE SILICONE 7207" par la Société UNION CARBIDE qsp | 100 g |

### EXEMPLE H

### Emulsion huile-dans-l'eau protectrice de l'épiderme humain

| | |
|---|---|
| - 2,4,-bis (4'-amino benzalmalonate de di 2-éthyl- hexyle)-6-(4'-amino benzoate de 2-éthylhexyle)-s-triazine (composé del'exemple 4) | 2,0 g |
| - Sel de triéthanolamine de l'acide benzène 1,4-[di(3-méthylidène camphométhyl sulfonique)] | 2,0 g |
| - Mélange d'alcool cétylstéarylique et cétylstéarylique oxyéthyléné à 33 moles d'oxyde d'éthylène ("SINNOWAX AO" vendu par la société HENKEL) | 7,0 g |
| - Monostéarate de glycérol | 2,0 g |
| - Propylène glycol | 10,0 g |
| - Alcool cétylique | 1,5 g |
| - Benzoate d'alcools C12/C15 ("FINSOLV TN" vendu par la société WITCO) | 15,0 g |
| - Conservateur | 0,2 g |
| - Parfum qs | |
| - Eau déminéralisée qsp | 100 g |

L'émulsion est préparée de la façon suivante :
On chauffe les corps gras et les émulsionnants vers 70-75°C; on ajoute le composé de l'exemple 4. Par ailleurs, on chauffe à 70-75°C l'eau contenant l'acide disulfonique neutralisé par la triéthanolamine et on ajoute la phase grasse à la phase aqueuse. Après 10 minutes d'agitation vive, on laisse refroidir sous agitation modérée et vers 40°C, on ajoute le conservateur et le parfum.

### EXEMPLE I

### Huile protectrice des cheveux

| | |
|---|---|
| - 2,4,6-tris[4'-amino benzalmalonate de di(2-éthylhexyle)-s-triazine (composé de l'exemple 3) | 1,0 g |
| -Alcool oléique | 19,5 g |
| - Hexylène glycol | 0,5 g |
| - Huile de colza qsp | 100 g |

Cette huile, d'aspect opalescent est appliquée sur cheveux séchés auxquels elle apporte de la brillance et de la douceur tout en préservant leur couleur lorsqu'ils sont exposés à la lumière naturelle.

### EXEMPLE J

### Crème de jour protectrice

| | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 7,0 g |
| - Mélange de mono- et distéarate de glycérol non autoémulsionnable | 2,0 g |
| - Alcool cétylique | 1,5 g |
| - Huile de silicone | 1,5 g |
| - Triglycérides d'acides caprique/caprylique vendus sous la dénomination commerciale de" MIGLYOL 812" par la Société DYNAMIT NOBEL | 20,0 g |
| - 2,4,6-tris (4'-amino benzalmalonate de diisobutyle)-s-triazine (exemple 1) | 1,0 g |
| - 4-tert.-butyl-4'-méthoxy dibenzoylméthane vendu sous la dénomination commerciale de "PARSOL 1789" par la Société GIVAUDAN | 1,0 g |
| - Glycérine | 20,0 g |
| - Parfum, conservateurs qs | |
| - Eau qsp | 100,0 g |

## Revendications (Revendications pour l'(les) Etat(s) contractant(s) suivant(s): AT, BE, CH, LI, DE, DK, FR, GB, GR, IT, NL, PT, SE)

1. Composé de formule : 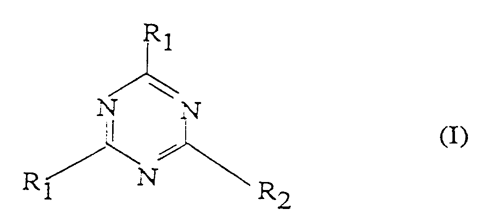 dans laquelle R₁ répond à la formule : 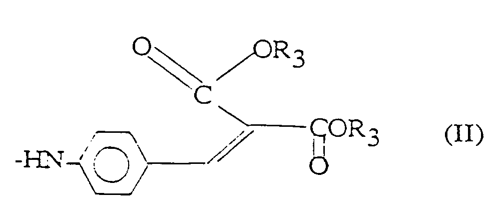 où R₃ représente un radical alkyle linéaire ou ramifié contenant de 1 à 20 atomes de carbone;
R₂ est identique à R₁ ou est un halogène, un groupement alcoxy ou mono- ou di-alkylamino linéaire ou ramifié contenant de 1 à 20 atomes de carbone ou un groupement répondant aux formules (III) ou (IV) ci-après : 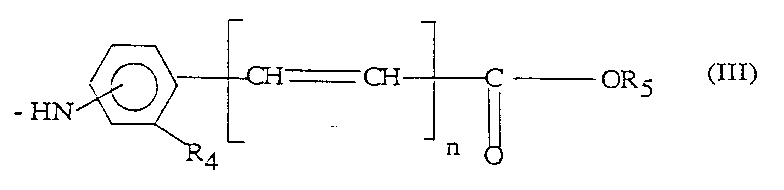 dans laquelle R₅ est un radical alkyle linéaire ou ramifié contenant de 1 à 20 atomes de carbone, et n = 0 ou 1;
- lorsque n est égal à 0, R₄ représente un atome d'hydrogène, le reste amino se trouvant en position 4 par rapport au groupement carboxyle, ou R₄ représente un radical hydroxyle ou un radical alcoxy en C₁-C₆, le reste amino se trouvant en position 4 ou 5 par rapport au groupement carboxyle;
- lorsque n est égal à 1, R₄ représente un atome d'hydrogène, le reste amino se trouvant en position 4 par rapport au groupement insaturé, 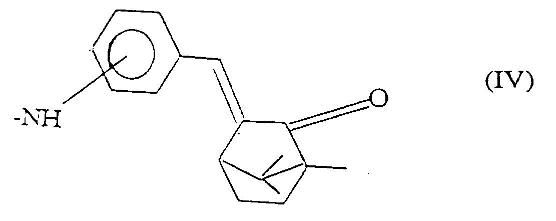 dans laquelle le reste amino se trouve en position 2, 3 ou 4 par rapport au groupement méthylidène camphre,
sous réserve que :
- lorsque R₂ est égal à R₁, R₃ est un radical comportant au moins 4 atomes de carbone;
- lorsque R₂ est différent de R₁ et répond à la formule (III), R₅ comporte au moins 4 atomes de carbone lorsque R₃ comporte de 1 à 7 atomes de carbone.
- lorsque R₂ est différent de R₁ et R₂ ne répond pas à la formule (III), mais est un radical alcoxy ou mono- ou di-alkylamino, R₂ comporte au moins 6 atomes de carbone lorsque R₃ comporte de 1 à 3 atomes de carbone.

2. Composé selon la revendication 1, caractérisé par le fait qu'il est choisi parmi les composés suivants : 2,4,6-tris(4'-amino benzalmalonate de diisobutyle)-s-triazine, 2,4-bis[4'-amino benzalmalonate de di(2-éthylhexyle)]-6-chloro-s-triazine, 2,4,6-tris[4'-amino benzalmalonate de di(2-éthylhexyle)]-s-triazine, 2,4-bis[(4'-amino benzalmalonate de di(2-éthylhexyle)]-6-(4'-amino benzoate de 2-éthylhexyle)-s-triazine, 2,4-bis(4'amino benzalmalonate de diisobutyle)-6-butoxy-s-triazine, 2,4-bis(4'-amino benzalmalonate de diisobutyle)-6-(2-éthylhexyl-amino)-s-triazine, 2,4-bis (4'-amino benzalmalonate de diisobutyle)-6-(5'-amino salicylate de 2-éthylhexyle)-s-triazine, 2,4-bis(4'-amino benzalmalonate de diisobutyle)-6-(4'-amino cinnamate de 2-éthylhexyle)-s-triazine, 2,4-bis(4'-aminobenzalmalonate de diisobutyle)-6-(4'-amino-benzylidène camphre)-s-triazine.

3. Procédé de préparation du composé de formule (I) selon la revendication 1 ou 2, caractérisé par le fait qu'il est mis en oeuvre selon le schéma réactionnel suivant : 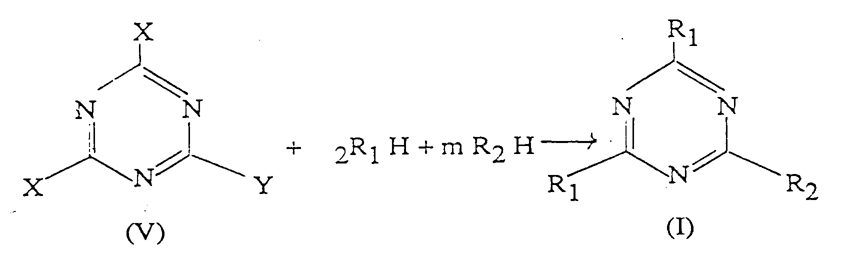 où R₁ répond à la formule (II) indiquée dans la revendication 1 et R₂ répond à la formule (II), (III) ou (IV) de la revendication 1; X représente un halogène; Y représente un halogène, un groupement alcoxy ou mono- ou di-alkylamino linéaire ou ramifié contenant de 1 à 20 atomes de carbone; m est un nombre entier égal à 1 si R₂ répond à la formule (II), (III) ou (IV) et est égal à O dans les autres cas,
la réaction étant effectuée éventuellement en présence d'un solvant tel que le toluène, le xylène ou un mélange acétone/eau, à une température comprise entre 0 et 250°C, de préférence entre 0 et 150°C.

4. Composition cosmétique caractérisée par le fait qu'elle comprend une quantité efficace d'au moins un composé de formule (I) selon la revendication 1 ou 2, dans un support cosmétiquement acceptable contenant au moins une phase grasse ou un solvant organique.

5. Composition cosmétique selon la revendication 4, caractérisée par le fait qu'elle comprend au moins un des composés choisis parmi les composés suivants :
2,4,6-tris(4'-amino benzalmalonate de diisobutyle)s-triazine, 2,4-bis[4'-amino benzalmalonate de di(2-éthylhexyle)]-6-chloro-s-triazine, 2,4,6-tris[4'-amino benzalmalonate de di(2-éthylhexyle)]-s-triazine, 2,4-bis[(4'-amino benzalmalonate de di(2-éthylhexyle)]-6-(4'-amino benzoate de 2-éthylhexyle)-s-triazine, 2,4-bis(4'amino benzalmalonate de diisobutyle)-6-butoxy-s-triazine, 2,4-bis(4'-amino benzal-malonate de diisobutyle)-6-(2-éthylhexyl-amino)-s-triazine, 2,4-bis (4'-amino benzalmalonate de diisobutyle)-6-(5'-amino salicylate de 2-éthylhexyle)-s-triazine, 2,4-bis(4'-amino benzalmalonate de diisobutyle)-6-(4'-aminocinnamate de 2-éthylhexyle)-s-triazine, 2,4-bis(4'-aminobenzalmalonate de diisobutyle)-6-(4'-amino-benzylidène camphre)-s-triazine.

6. Composition cosmétique selon la revendication 4 ou 5, caractérisée par le fait qu'elle se présente sous forme de lotion huileuse ou oléoalcoolique, d'émulsion, de dispersion vésiculaire de lipides amphiphiles ioniques ou non-ioniques, de gel oléoalcoolique ou alcoolique, de bâtonnet solide ou d'aérosol.

7. Composition cosmétique selon la revendication 6, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, propulseurs, colorants et pigments.

8. Composition cosmétique protectrice de l'épiderme humain selon l'une quelconque des revendications 4 à 7, caractérisée par le fait qu'elle contient 0,1 à 2% en poids de composé de formule (I)

9. Composition cosmétique anti-solaire selon l'une quelconque des revendications 4 à 7, caractérisée par le fait qu'elle contient 0,3 à 15% en poids de composé de formule (I).

10. Composition cosmétique anti-solaire selon la revendication 9, caractérisée par le fait qu'elle contient en outre un agent filtrant les rayons UV-B et/ou UV-A différent du composé de formule (I).

11. Composition cosmétique selon la revendication 4 ou 5, destinée à être appliquée sur les cheveux, caractérisée par le fait qu'elle se présente sous forme de shampooing, lotion, gel ou émulsion à rincer, lotion ou gel coiffants ou traitants, lotion ou gel pour le brushing ou la mise en plis, laque pour cheveux, composition de permanente, de décoloration ou coloration et comprend 0,1 à 2%, en poids de composé de formule (I).

12. Composition cosmétique selon la revendication 4 ou 5, sous forme d'une composition cosmétique stabilisée à la lumière, caractérisée par le fait qu'elle est constituée par une composition capillaire, un produit de maquillage ou une composition pour les soins ou pour le traitement de la peau, comprenant 0,1 à 2% en poids de composé de formule (I).

13. Procédé non-thérapeutique de protection de la peau et des cheveux naturels ou sensibilisés contre le rayonnement ultraviolet, caractérisé par le fait qu'il consiste à appliquer sur la peau ou les cheveux une quantité efficace d'une composition cosmétique contenant au moins un composé de formule (I) selon la revendication 1 ou 2.

## Revendications (Revendications pour l'(les) Etat(s) contractant(s) suivant(s): ES)

1. Procédé de préparation du composé de formule (I) suivante : 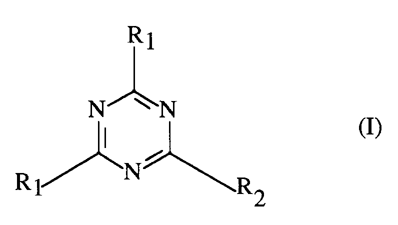 dans laquelle R₁ répond à la formule : 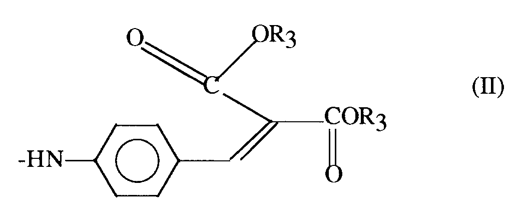 où R₃ représente un radical alkyle linéaire ou ramifié contenant de 1 à 20 atomes de carbone;
R₂ est identique à R₁ ou est un halogène, un groupement alcoxy ou mono- ou di-alkylamino linéaire ou ramifié contenant de 1 à 20 atomes de carbone ou un groupement répondant aux formules (III) ou (IV) ci-après : 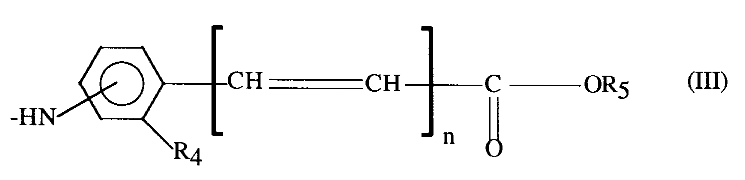 dans laquelle R₅ est un radical alkyle linéaire ou ramifié contenant de 1 à 20 atomes de carbone, et n = 0 ou 1;
- lorsque n est égal à 0, R₄ représente un atome d'hydrogène, le reste amino se trouvant en position 4 par rapport au groupement carboxyle, ou R₄ représente un radical hydroxyle ou un radical alcoxy en C₁-C₆, le reste amino se trouvant en position 4 ou 5 par rapport au groupement carboxyle;
- lorsque n est égal à 1, R₄ représente un atome d'hydrogène, le reste amino se trouvant en position 4 par rapport au groupement insaturé, 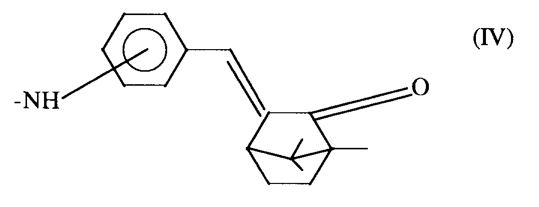 dans laquelle le reste amino se trouve en position 2, 3 ou 4 par rapport au groupement méthylidène camphre,
sous réserve que :
- lorsque R₂ est égal à R₁, R₃ est un radical comportant au moins 4 atomes de carbone;
- lorsque R₂ est différent de R₁ et répond à la formule (III), R₅ comporte au moins 4 atomes de carbone lorsque R₃ comporte de 1 à 7 atomes de carbone.
- lorsque R₂ est différent de R₁ et R₂ ne répond pas à la formule (III), mais est un radical alcoxy ou mono- ou di-alkylamino, R₂ comporte au moins 6 atomes de carbone lorsque R₃ comporte de 1 à 3 atomes de carbone,
caractérisé par le fait qu'il est mis en oeuvre selon le schéma réactionnel suivant : 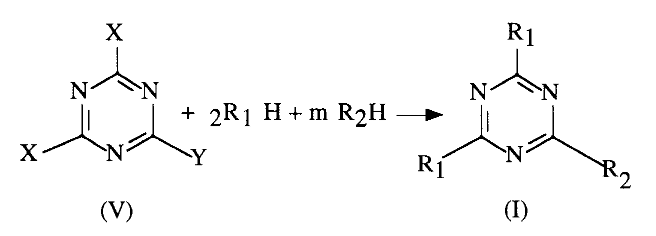 où R₁ répond à la formule (II) indiquée ci-dessus et R₂ répond à la formule (II), (III) ou (IV) ci-dessus; X représente un halogène; Y représente un halogène, un groupement alcoxy ou mono- ou dialkylamino linéaire ou ramifié contenant de 1 à 20 atomes de carbone; m est un nombre entier égal à 1 si R₂ répond à la formule (II), (III) ou (IV) et est égal à O dans les autres cas,
la réaction étant effectuée éventuellement en présence d'un solvant tel que le toluène, le xylène ou un mélange acétone/eau, à une température comprise entre 0 et 250°C, de préférence entre 0 et 150°C.

2. Procédé de préparation du composé de formule (I) selon la revendication 1, caractérisé par le fait qu'il consiste à préparer l'un des composés suivants :
2,4,6-tris(4'-amino benzalmalonate de diisobutyle)-s-triazine, 2,4-bis[4'-amino benzalmalonate de di(2-éthylhexyle)]-6-chloro-s-triazine, 2,4,6-tris[4'-amino benzalmalonate de di(2-éthylhexyle)]-s-triazine, 2,4-bis[(4'-amino benzalmalonate de di(2-éthylhexyle)]-6-(4'-amino benzoate de 2-éthylhexyle)-s-triazine, 2,4-bis(4'amino benzalmalonate de diisobutyle)-6-butoxy-s-triazine, 2,4-bis(4'-amino benzalmalonate de diisobutyle)-6-(2-éthylhexyl-amino)-s-triazine, 2,4-bis (4'-amino benzalmalonate de diisobutyle)-6-(5'-amino salicylate de 2-éthylhexyle)-s-triazine, 2,4-bis(4'-amino benzalmalonate de diisobutyle)-6-(4'-amino cinnamate de 2-éthylhexyle)-s-triazine, 2,4-bis(4'-aminobenzalmalonate de diisobutyle)-6-(4'-amino-benzylidène camphre)-s-triazine.

3. Composition cosmétique caractérisée par le fait qu'elle comprend, dans un support cosmétiquement acceptable contenant au moins une phase grasse ou un solvant organique, une quantité efficace d'au moins un composé de formule (I) suivante : 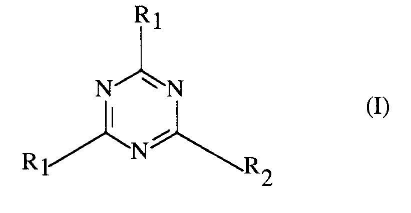 dans laquelle R₁ répond à la formule : 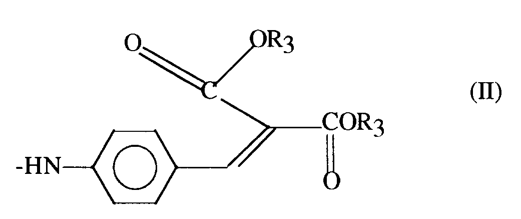 où R₃ représente un radical alkyle linéaire ou ramifié contenant de 1 à 20 atomes de carbone;
R₂ est identique à R₁ ou est un halogène, un groupement alcoxy ou mono- ou di-alkylamino linéaire ou ramifié contenant de 1 à 20 atomes de carbone ou un groupement répondant aux formules (III) ou (IV) ci-après : 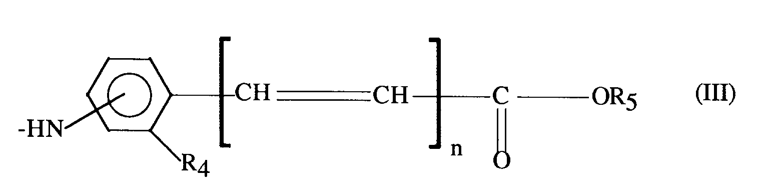 dans laquelle R₅ est un radical alkyle linéaire ou ramifié contenant de 1 à 20 atomes de carbone, et n = 0 ou 1;
- lorsque n est égal à 0, R₄ représente un atome d'hydrogène, le reste amino se trouvant en position 4 par rapport au groupement carboxyle, ou R₄ représente un radical hydroxyle ou un radical alcoxy en C₁-C₆, le reste amino se trouvant en position 4 ou 5 par rapport au groupement carboxyle;
- lorsque n est égal à 1, R₄ représente un atome d'hydrogène, le reste amino se trouvant en position 4 par rapport au groupement insaturé, 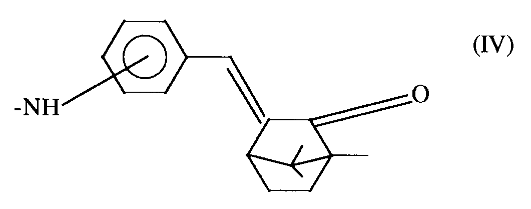 dans laquelle le reste amino se trouve en position 2, 3 ou 4 par rapport au groupement méthylidène camphre,
sous réserve que :
- lorsque R₂ est égal à R₁, R₃ est un radical comportant au moins 4 atomes de carbone;
- lorsque R₂ est différent de R₁ et répond à la formule (III), R₅ comporte au moins 4 atomes de carbone lorsque R₃ comporte de 1 à 7 atomes de carbone.
- lorsque R₂ est différent de R₁ et R₂ ne répond pas à la formule (III), mais est un radical alcoxy ou mono- ou di-alkylamino, R₂ comporte au moins 6 atomes de carbone lorsque R₃ comporte de 1 à 3 atomes de carbone.

4. Composition cosmétique selon la revendication 3, caractérisée par le fait qu'elle comprend au moins un des composés choisis parmi les composés suivants :
2,4,6-tris(4'-amino benzalmalonate de diisobutyle)-s-triazine, 2,4-bis[4'-amino benzalmalonate de di(2-éthylhexyle)]-6-chloro-s-triazine, 2,4,6-tris[4'-amino benzalmalonate de di(2-éthylhexyle)]-s-triazine, 2,4-bis[(4'-amino benzalmalonate de di(2-éthylhexyle)]-6-(4'-amino benzoate de 2-éthylhexyle)-s-triazine, 2,4-bis(4'amino benzalmalonate de diisobutyle)-6-butoxy-s-triazine, 2,4-bis(4'-amino benzal-malonate de diisobutyle)-6-(2-éthylhexyl-amino)-s-triazine, 2,4-bis (4'-amino benzalmalonate de diisobutyle)-6-(5'amino salicylate de 2-éthylhexyle)-s-triazine, 2,4-bis(4'-amino benzalmalonate de diisobutyle)-6-(4'-aminocinnamate de 2-éthylhexyle)-s-triazine, 2,4-bis(4'-aminobenzalmalonate de diisobutyle)-6-(4'-amino-benzylidène camphre)-s-triazine.

5. Composition cosmétique selon la revendication 3 ou 4, caractérisée par le fait qu'elle se présente sous forme de lotion huileuse ou oléoalcoolique, d'émulsion, de dispersion vésiculaire de lipides amphiphiles ioniques ou non-ioniques, de gel oléoalcoolique ou alcoolique, de bâtonnet solide ou d'aérosol.

6. Composition cosmétique selon la revendication 5, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, propulseurs, colorants et pigments.

7. Composition cosmétique protectrice de l'épiderme humain selon l'une quelconque des revendications 3 à 6, caractérisée par le fait qu'elle contient 0,1 à 2% en poids de composé de formule (I)

8. Composition cosmétique anti-solaire selon l'une quelconque des revendications 3 à 6, caractérisée par le fait qu'elle contient 0,3 à 15% en poids de composé de formule (I).

9. Composition cosmétique anti-solaire selon la revendication 8, caractérisée par le fait qu'elle contient en outre un agent filtrant les rayons UV-B et/ou UV-A différent du composé de formule (I).

10. Composition cosmétique selon la revendication 3 ou 4, destinée à être appliquée sur les cheveux, caractérisée par le fait qu'elle se présente sous forme de shampooing, lotion, gel ou émulsion à rincer, lotion ou gel coiffants ou traitants, lotion ou gel pour le brushing ou la mise en plis, laque pour cheveux, composition de permanente, de décoloration ou coloration et comprend 0,1 à 2%, en poids de composé de formule (I).

11. Composition cosmétique selon la revendication 3 ou 4, sous forme d'une composition cosmétique stabilisée à la lumière, caractérisée par le fait qu'elle est constituée par une composition capillaire, un produit de maquillage ou une composition pour les soins ou pour le traitement de la peau, comprenant 0,1 à 2% en poids de composé de formule (I).

12. Procédé de préparation d'une composition cosmétique, caractérisé par le fait qu'il consiste à incorporer 0,1 à 15% en poids d'un composé de formule (I) suivante : 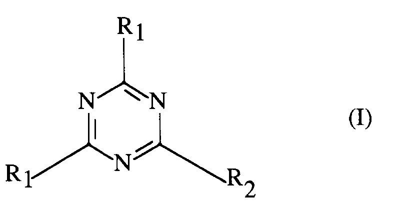 dans laquelle R₁ répond à la formule : 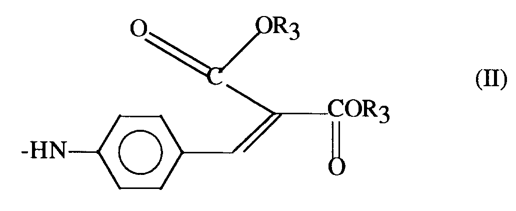 où R₃ représente un radical alkyle linéaire ou ramifié contenant de 1 à 20 atomes de carbone;
R₂ est identique à R₁ ou est un halogène, un groupement alcoxy ou mono- ou di-alkylamino linéaire ou ramifié contenant de 1 à 20 atomes de carbone ou un groupement répondant aux formules (III) ou (IV) ci-après : 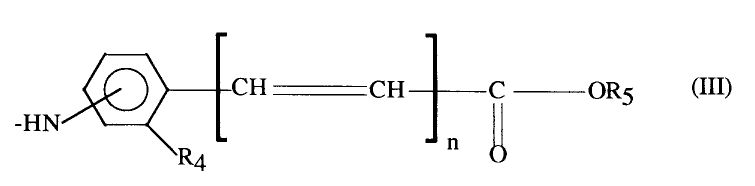 dans laquelle R₅ est un radical alkyle linéaire ou ramifié contenant de 1 à 20 atomes de carbone, et n = 0 ou 1;
- lorsque n est égal à 0, R₄ représente un atome d'hydrogène, le reste amino se trouvant en position 4 par rapport au groupement carboxyle, ou R₄ représente un radical hydroxyle ou un radical alcoxy en C₁-C₆, le reste amino se trouvant en position 4 ou 5 par rapport au groupement carboxyle;
- lorsque n est égal à 1, R₄ représente un atome d'hydrogène, le reste amino se trouvant en position 4 par rapport au groupement insaturé, 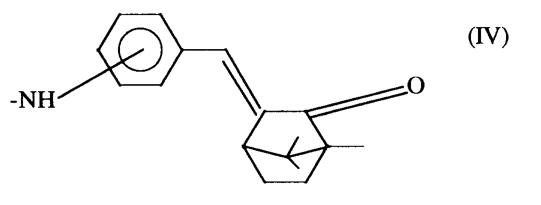 dans laquelle le reste amino se trouve en position 2, 3 ou 4 par rapport au groupement méthylidène camphre,
sous réserve que :
- lorsque R₂ est égal à R₁, R₃ est un radical comportant au moins 4 atomes de carbone;
- lorsque R₂ est différent de R₁ et répond à la formule (III), R₅ comporte au moins 4 atomes de carbone lorsque R₃ comporte de 1 à 7 atomes de carbone;
- lorsque R₂ est différent de R₁ et R₂ ne répond pas à la formule (III), mais est un radical alcoxy ou mono- ou di-alkylamino, R₂ comporte au moins 6 atomes de carbone lorsque R₃ comporte de 1 à 3 atomes de carbone,
dans un support cosmétiquement acceptable comprenant au moins une phase grasse ou un solvant organique choisi parmi les huiles, les cires, les corps gras, les monoalcools et polyols inférieurs et leurs mélanges.

## Claims (Claims for the following Contracting State(s): AT, BE, CH, LI, DE, DK, FR, GB, GR, IT, NL, PT, SE)

1. Compound of formula: 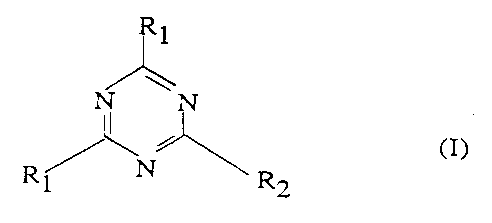 in which R₁ is of the formula: 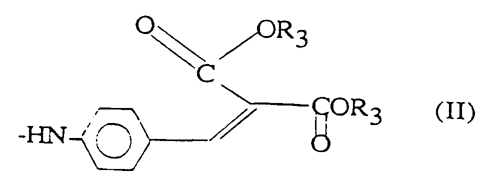 where R₃ represents a linear or branched alkyl radical containing 1 to 20 carbon atoms;
R₂ is identical to R₁, or is a halogen, a linear or branched alkoxy or mono- or dialkylamino group containing 1 to 20 carbon atoms or a group of the formulae (III) or (IV) below: 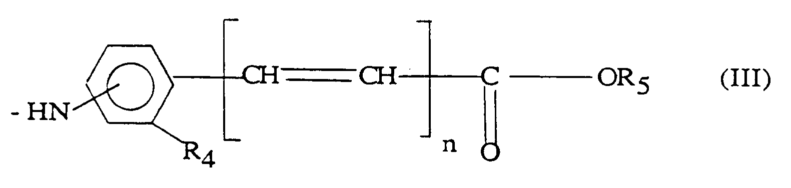 in which R₅ is a linear or branched alkyl radical containing 1 to 20 carbon atoms, and n = 0 or 1;
- when n is equal to 0, R₄ represents a hydrogen atom, the amino residue being in position 4 with respect to the carboxyl group, or R₄ represents a hydroxyl radical or a C₁-C₆ alkoxy radical, the amino residue being in position 4 or 5 with respect to the carboxyl group;
- when n is equal to 1, R₄ represents a hydrogen atom, the amino residue being in position 4 with respect to the unsaturated group, 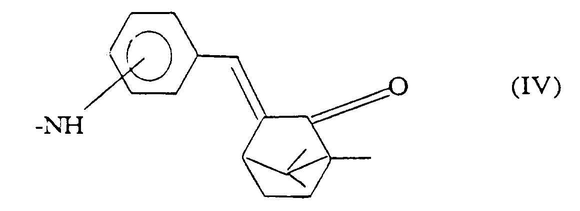 in which the amino residue is in position 2, 3 or 4 with respect to the methylidenecamphor group,
provided that:
- when R₂ is equal to R₁, R₃ is a radical containing not less than 4 carbon atoms;
- when R₂ is different from R₁ and is of the formula (III), R₅ contains not less than 4 carbon atoms when R₃ contains 1 to 7 carbon atoms;
- when R₂ is different from R₁ and R₂ is not of the formula (III), but is an alkoxy or a mono- or dialkylamino radical, R₂ contains not less than 6 carbon atoms when R₃ contains 1 to 3 carbon atoms.

2. Compound according to Claim 1, characterised in that it is chosen from the following compounds: 2,4,6-tris(diisobutyl 4'-aminobenzalmalonate)-s-triazine, 2,4-bis[di(2-ethylhexyl) 4'-aminobenzalmalonate]-6-chloro-s-triazine, 2,4,6-tris[di(2-ethylhexyl) 4'-aminobenzalmalonate]-s-triazine, 2,4-bis[di(2-ethylhexyl) 4'-aminobenzalmalonate]-6-(2-ethylhexyl 4'-aminobenzoate)-s-triazine, 2,4-bis(diisobutyl 4'-aminobenzalmalonate)-6-butoxy-s-triazine, 2,4-bis(diisobutyl 4'-aminobenzalmalonate)-6-(2-ethylhexylamino)-s-triazine, 2,4-bis(diisobutyl 4'-aminobenzalmalonate)-6-(2-ethylhexyl 5'-aminosalicylate)-s-triazine, 2,4-bis(diisobutyl 4'-aminobenzalmalonate)-6-(2-ethylhexyl 4'-aminocinnamate)-s-triazine,2,4-bis(diisobutyl-4'-aminobenzalmalonate)-6-(4'-aminobenzylidenecamphor)-s-triazine.

3. Method of preparing the compound of formula (I) according to Claim 1 or 2, characterised in that it is obtained according to the following reaction scheme: 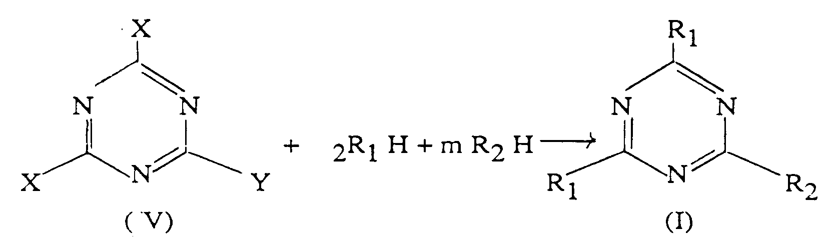 where R₁ is of the formula (II) indicated in Claim 1 and R₂ is of the formula (II), (III) or (IV) of Claim 1; X represents a halogen; Y represents a halogen, a linear or branched alkoxy or mono- or dialkylamino group containing 1 to 20 carbon atoms; m is an integer equal to 1 if R₂ is of the formula (II), (III) or (IV) and is equal to 0 in the other cases,
the reaction being optionally carried out in the presence of a solvent such as toluene, xylene or an acetone/water mixture, at a temperature between 0 and 250°C, preferably between 0 and 150°C.

4. Cosmetic composition characterised in that it contains an effective amount of at least one compound of formula (I) according to Claim 1 or 2, in a cosmetically acceptable carrier containing at least one fatty phase or one organic solvent.

5. Cosmetic composition according to Claim 4, characterised in that it contains at least one compound chosen from the following compounds: 2,4,6-tris(diisobutyl 4'-aminobenzalmalonate)-s-triazine, 2,4-bis[di(2-ethylhexyl) 4'-aminobenzalmalonate]-6-chloro-s-triazine, 2,4,6-tris[di(2-ethylhexyl) 4'-aminobenzalmalonate]-s-triazine, 2,4-bis[di(2-ethylhexyl) 4'-aminobenzalmalonate]-6-(2-ethylhexyl 4'-aminobenzoate)-s-triazine, 2,4-bis(diisobutyl 4'-aminobenzalmalonate)-6-butoxy-s-triazine, 2,4-bis(diisobutyl 4'-aminobenzalmalonate)-6-(2-ethylhexylamino)-s-triazine, 2,4-bis(diisobutyl 4'-aminobenzalmalonate)-6-(2-ethylhexyl 5'-aminosalicylate)-s-triazine, 2,4-bis(diisobutyl 4'-aminobenzalmalonate)-6-(2-ethylhexyl 4'-aminocinnamate)-s-triazine, 2,4-bis(diisobutyl 4'-aminobenzalmalonate)-6-(4'-aminobenzylidenecamphor)-s-triazine.

6. Cosmetic composition according to Claim 4 or 5, characterised in that it is provided in the form of an oily or oil-alcohol lotion, emulsion, vesicular dispersion of ionic or nonionic amphiphilic lipids, oil-alcohol or alcoholic gel, solid stick or aerosol.

7. Cosmetic composition according to Claim 6, characterised in that it contains in addition cosmetic adjuvants chosen from thickeners, demulcents, humectants, surface-active agents, preservatives, antifoams, perfumes, oils, waxes, lanolin, lower monoalcohols and polyols, propellants, colorants and pigments.

8. Cosmetic human epidermis-protecting composition according to any one of Claims 4 to 7, characterised in that it contains 0.1 to 2 % by weight of the compound of formula (I).

9. Cosmetic antisun composition according to any one of Claims 4 to 7, characterised in that it contains 0.3 to 15 % by weight of the compound of formula (I).

10. Cosmetic antisun composition according to Claim 9, characterised in that it contains in addition an agent screening out UV-B and/or UV-A rays which is different from the compound of formula (I).

11. Cosmetic composition according to Claim 4 or 5, intended to be applied to hair, characterised in that it is provided in the form of a shampoo, rinse off lotion, gel or emulsion, hair styling or treating lotion or gel, blow drying or hair setting lotion or gel, hair lacquer, permanent wave, bleaching or dyeing composition, and contains 0.1 to 2 % by weight of the compound of formula (I).

12. Cosmetic composition according to Claim 4 or 5, in the form of a cosmetic composition stabilised with respect to light, characterised in that it consists of a hair composition, a make up product or a skin care or treatment composition, containing 0.1 to 2 % by weight of the compound of formula (I).

13. A non-therapeutic method of protecting the skin and natural or sensitised hair against ultraviolet radiation , characterised in that it consists in applying to the skin or the hair an effective amount of a cosmetic composition containing at least one compound of formula (I) according to Claim 1 or 2.

## Claims (Claims for the following Contracting State(s): ES)

1. Process for preparing the compound of the following formula (I): 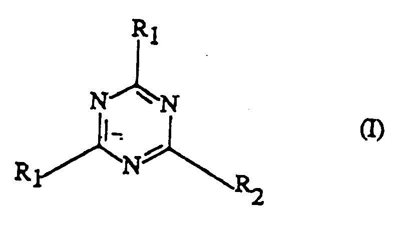 in which R₁ is of the formula: 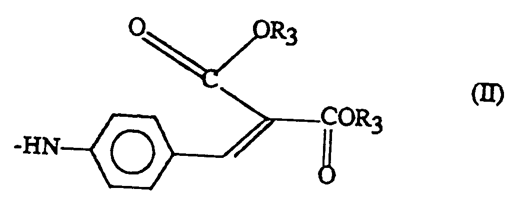 where R₃ represents a linear or branched alkyl radical containing 1 to 20 carbon atoms;
R₂ is identical to R₁, or is a halogen, a linear or branched alkoxy or mono- or dialkylamino group containing 1 to 20 carbon atoms or a group of the formulae (III) or (IV) below: 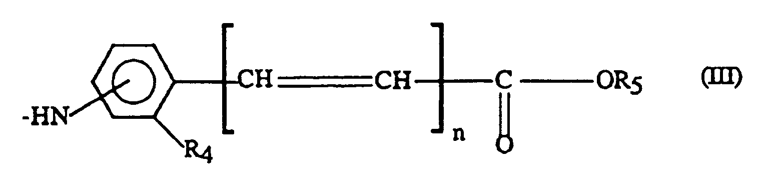 in which R₅ is a linear or branched alkyl radical containing 1 to 20 carbon atoms, and n = 0 or 1;
- when n is equal to 0, R₄ represents a hydrogen atom, the amino residue being in position 4 with respect to the carboxyl group, or R₄ represents a hydroxyl radical or a C₁-C₆ alkoxy radical, the amino residue being in position 4 or 5 with respect to the carboxyl group;
- when n is equal to 1, R₄ represents a hydrogen atom, the amino residue being in position 4 with respect to the unsaturated group, 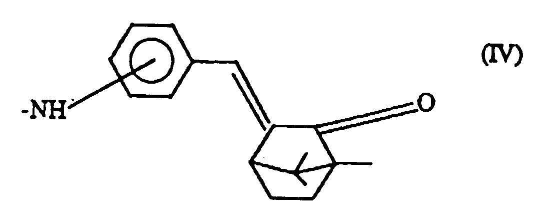 in which the amino residue is in position 2, 3 or 4 with respect to the methylidenecamphor group,
provided that:
- when R₂ is equal to R₁, R₃ is a radical containing not less than 4 carbon atoms;
- when R₂ is different from R₁ and is of the formula (III), R₅ contains not less than 4 carbon atoms when R₃ contains 1 to 7 carbon atoms;
- when R₂ is different from R, and R₂ is not of the formula (III), but is an alkoxy or a mono- or dialkylamino radical, R₂ contains not less than 6 carbon atoms when R₃ contains 1 to 3 carbon atoms,
characterised in that it is implemented according to the following reaction scheme: 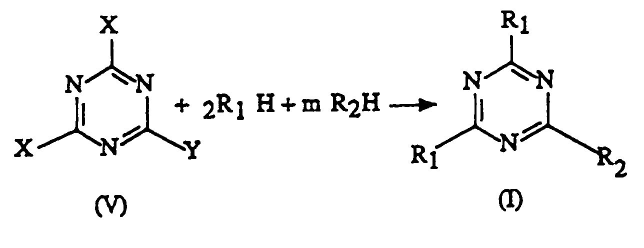 where R₁ is of the formula (II) indicated above and R₂ is of the formula (II), (III) or (IV) above; X represents a halogen; Y represents a halogen, a linear or branched alkoxy or mono- or dialkylamino group containing 1 to 20 carbon atoms; m is an integer equal to 1 if R₂ is of the formula (II), (III) or (IV) and is equal to 0 in the other cases,
the reaction being optionally carried out in the presence of a solvent such as toluene, xylene or an acetone/water mixture, at a temperature between 0 and 250°C, preferably between 0 and 150°C.

2. Process for preparing the compound of formula (I) according to Claim 1, characterised in that it consists in preparing one of the following compounds:
2,4,6-tris(diisobutyl 4'-aminobenzalmalonate)-s-triazine, 2,4-bis[di(2-ethylhexyl) 4'-aminobenzalmalonate]-6-chloro-s-triazine, 2,4,6-tris[di(2-ethylhexyl) 4'-aminobenzalmalonate]-s-triazine, 2,4-bis[di(2-ethylhexyl) 4'-aminobenzalmalonate]-6-(2-ethylhexyl 4'-aminobenzoate)-s-triazine, 2,4-bis(diisobutyl 4'-aminobenzalmalonate)-6-butoxy-s-triazine, 2,4-bis(diisobutyl 4'-aminobenzalmalonate)-6-(2-ethylhexylamino)-s-triazine, 2,4-bis(diisobutyl 4'-aminobenzalmalonate)-6-(2-ethylhexyl 5'-aminosalicylate)-s-triazine, 2,4-bis(diisobutyl 4'-aminobenzalmalonate)-6-(2-ethylhexyl 4'-aminocinnamate)-s-triazine, 2,4-bis(diisobutyl 4'-aminobenzalmalonate)-6-(4'-aminobenzylidenecamphor)-s-triazine.

3. Cosmetic composition characterised in that it contains, in a cosmetically acceptable carrier containing at least one fatty phase or one organic solvent, an effective amount of at least one compound of the following formula (I): 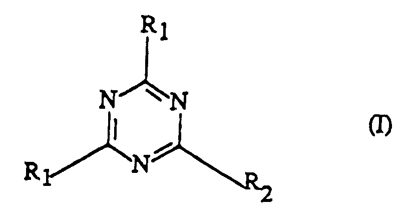 in which R₁ is of the formula: 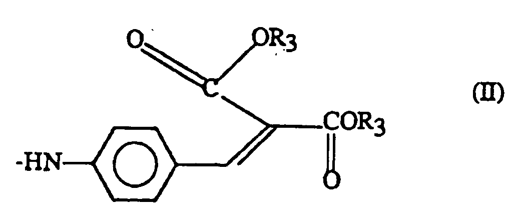 where R₃ represents a linear or branched alkyl radical containing 1 to 20 carbon atoms;
R₂ is identical to R₁, or is a halogen, a linear or branched alkoxy or mono- or dialkylamino group containing 1 to 20 carbon atoms or a group of the formulae (III) or (IV) below: 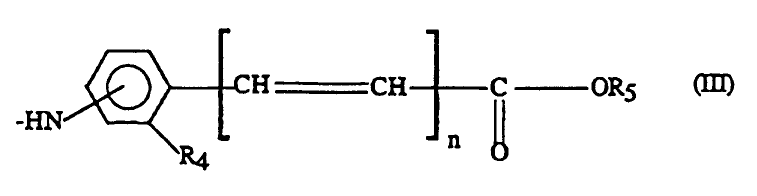 in which R₅ is a linear or branched alkyl radical containing 1 to 20 carbon atoms, and n = 0 or 1;
- when n is equal to 0, R₄ represents a hydrogen atom, the amino residue being in position 4 with respect to the carboxyl group, or R₄ represents a hydroxyl radical or a C₁-C₆ alkoxy radical, the amino residue being in position 4 or 5 with respect to the carboxyl group;
- when n is equal to 1, R₄ represents a hydrogen atom, the amino residue being in position 4 with respect to the unsaturated group, 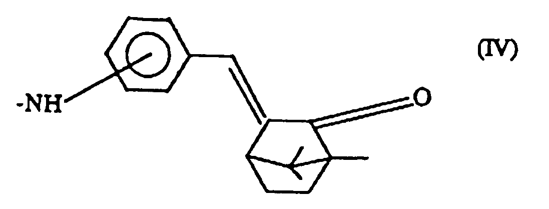 in which the amino residue is in position 2, 3 or 4 with respect to the methylidenecamphor group,
provided that:
- when R₂ is equal to R₁, R₃ is a radical containing not less than 4 carbon atoms;
- when R₂ is different from R₁ and is of the formula (III), R₅ contains not less than 4 carbon atoms when R₃ contains 1 to 7 carbon atoms;
- when R₂ is different from R₁ and R₂ is not of the formula (III), but is an alkoxy or a mono- or dialkylamino radical, R₂ contains not less than 6 carbon atoms when R₃ contains 1 to 3 carbon atoms.

4. Cosmetic composition according to Claim 3, characterised in that it contains at least one compound chosen from the following compounds: 2,4,6-tris(diisobutyl 4'-aminobenzalmalonate)-s-triazine, 2,4-bis[di(2-ethylhexyl) 4'-aminobenzalmalonate]-6-chloro-s-triazine, 2,4,6-tris[di(2-ethylhexyl) 4'-aminobenzalmalonate]-s-triazine, 2,4-bis[di(2-ethylhexyl) 4'-aminobenzalmalonate]-6-(2-ethylhexyl 4'-aminobenzoate)-s-triazine, 2,4-bis(diisobutyl 4'-aminobenzalmalonate)-6-butoxy-s-triazine, 2,4-bis(diisobutyl 4'-aminobenzalmalonate)-6-(2-ethylhexylamino)-s-triazine, 2,4-bis(diisobutyl 4'-aminobenzalmalonate)-6-(2-ethylhexyl 5'-aminosalicylate)-s-triazine, 2,4-bis(diisobutyl 4'-aminobenzalmalonate)-6-(2-ethylhexyl 4'-aminocinnamate)-s-triazine, 2,4-bis(diisobutyl 4'-aminobenzalmalonate)-6-(4'-aminobenzylidenecamphor)-s-triazine.

5. Cosmetic composition according to Claim 3 or 4, characterised in that it is provided in the form of an oily or oil-alcohol lotion, emulsion, vesicular dispersion of ionic or nonionic amphiphilic lipids, oil-alcohol or alcoholic gel, solid sticks or aerosol.

6. Cosmetic composition according to Claim 5, characterised in that it contains in addition cosmetic adjuvants chosen from thickeners, demulcents, humectants, surface-active agents, preservatives, antifoams, perfumes, oils, waxes, lanolin, lower monoalcohols and polyols, propellants, colorants and pigments.

7. Cosmetic human epidermis-protecting composition according to any one of Claims 3 to 6, characterised in that it contains 0.1 to 2 % by weight of the compound of formula (I).

8. Cosmetic antisun composition according to any one of Claims 3 to 6, characterised in that it contains 0.3 to 15 % by weight of the compound of formula (I).

9. Cosmetic antisun composition according to Claim 8, characterised in that it contains in addition an agent screening out UV-B and/or UV-A rays which is different from the compound of formula (I).

10. Cosmetic composition according to Claim 3 or 4, intended to be applied to hair, characterised in that it is provided in the form of a shampoo, rinse off lotion, gel or emulsion, hair styling or treating lotion or gel, blow drying or hair setting lotion or gel, hair lacquer, permanent wave, bleaching or dyeing composition, and contains 0.1 to 2 % by weight of the compound of formula (I).

11. Cosmetic composition according to Claim 3 or 4, in the form of a cosmetic composition stabilised with respect to light, characterised in that it consists of a hair composition, a make up product or a skin care or treatment composition, containing 0.1 to 2 % by weight of the compound of formula (I).

12. Process for preparing a cosmetic composition, characterised in that it consists in incorporating 0.1 to 15% by weight of a compound of the following formula (I): 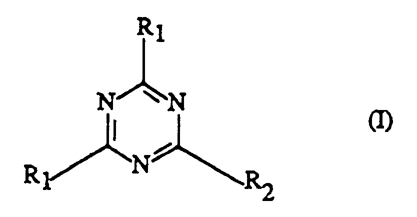 in which R₁ is of the formula: 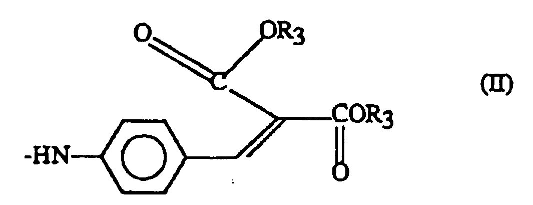 where R₃ represents a linear or branched alkyl radical containing 1 to 20 carbon atoms;
R₂ is identical to R₁, or is a halogen, a linear or branched alkoxy or mono- or dialkylamino group containing 1 to 20 carbon atoms or a group of the formulae (III) or (IV) below: 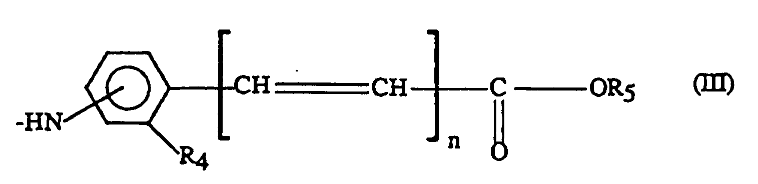 in which R₅ is a linear or branched alkyl radical containing 1 to 20 carbon atoms, and n = 0 or 1;
- when n is equal to 0, R₄ represents a hydrogen atom, the amino residue being in position 4 with respect to the carboxyl group, or R₄ represents a hydroxyl radical or a C₁-C₆ alkoxy radical, the amino residue being in position 4 or 5 with respect to the carboxyl group;
- when n is equal to 1, R₄ represents a hydrogen atom, the amino residue being in position 4 with respect to the unsaturated group, 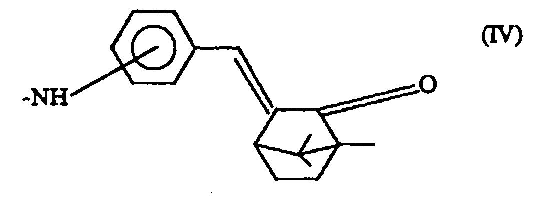 in which the amino residue is in position 2, 3 or 4 with respect to the methylidenecamphor group,
provided that:
- when R₂ is equal to R₁, R₃ is a radical containing not less than 4 carbon atoms;
- when R₂ is different from R₁ and is of the formula (III), R₅ contains not less than 4 carbon atoms when R₃ contains 1 to 7 carbon atoms;
- when R₂ is different from R₁ and R₂ is not of the formula (III), but is an alkoxy or a mono- or dialkylamino radical, R₂ contains not less than 6 carbon atoms when R₃ contains 1 to 3 carbon atoms,
in a cosmetically acceptable carrier containing at least one fatty phase or one organic solvent chosen from oils, waxes, fatty substances, lower monoalcohols and polyols and mixtures thereof.

## Patentansprüche (Patentansprüche für folgende(n) Vertragsstaat(en): AT, BE, CH, LI, DE, DK, FR, GB, GR, IT, NL, PT, SE)

1. Verbindung der Formel 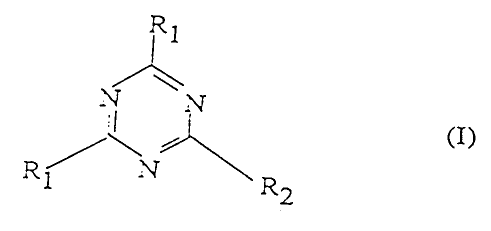 worin R₁ die Formel aufweist: 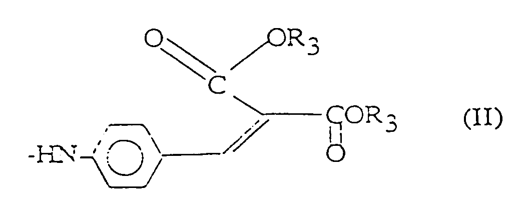 in welcher R₃ einen linearen oder verzweigten Alkylrest mit 1 bis 20 Kohlenstoffatomen darstellt,
und worin R₂ gleich R₁ ist oder ein Halogenatom, eine lineare oder verzweigte Alkoxy- oder Mono- oder Dialkylaminogruppe mit 1 bis 20 Kohlenstoffatomen oder eine Gruppe der folgenden Formel (III) oder der weiter unten angegebenen Formel (IV) darstellt: 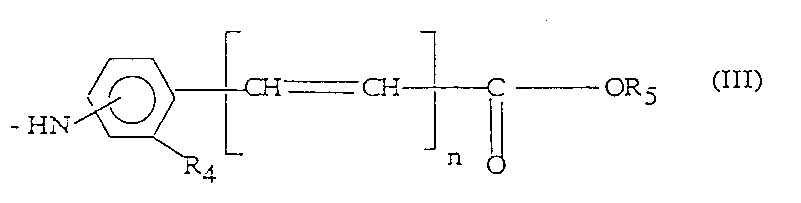 worin R₅ ein linearer oder verzweigter Alkylrest mit 1 bis 20 Kohlenstoffatomen ist und n = 0 oder 1, wobei gilt:
- wenn n gleich 0 ist, stellt R₄ ein Wasserstoffatom dar, wobei der Aminorest in Position 4 bezüglich der Carboxylgruppe vorliegt, oder stellt R₄ einen Hydroxylrest oder einen C₁₋₆-Alkoxyrest dar, wobei der Aminorest in Position 4 oder 5 bezüglich der Carboxylgruppe vorliegt;
- wenn n gleich 1 ist, stellt R₄ ein Wasserstoffatom dar, wobei der Aminorest in Position 4 bezüglich der ungesättigten Gruppierung vorliegt, 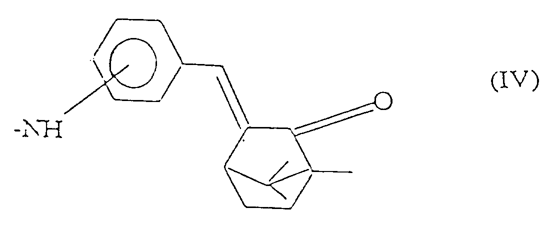 worin der Aminorest in Position 2, 3 oder 4 bezüglich der Methylidencamphergruppierung vorliegt,
mit der Maßgabe, daß gilt:
- wenn R₂ gleich R₁ ist, stellt R₃ einen Rest mit mindestens 4 Kohlenstoffatomen dar;
- wenn R₂ von R₁ verschieden ist und die Formel (III) aufweist, enthält R₅ mindestens 4 Kohlenstoffatome, wenn R₃ 1 bis 7 Kohlenstoffatome enthält;
- wenn R₂ verschieden von R₁ ist und nicht die Formel (III) aufweist, aber ein Alkoxy- oder Mono- oder Dialkylaminorest ist, weist R₂ mindestens 6 Kohlenstoffatome auf, wenn R₃ 1 bis 3 Kohlenstoffatome enthält.

2. Verbindung gemäß Anspruch 1,
dadurch **gekennzeichnet,** daß
sie aus den folgenden Verbindungen ausgewählt ist:
1) 2,4,6-Tris(4'-aminobenzaldiisobutylmalonat)-s-triazin
2) 2,4-Bis(4'-aminobenzaldi(2-ethylhexyl)malonat)-6-chlor-s-triazin
3) 2,4,6-Tris(4'-aminobenzaldi(2-ethylhexyl)malonat)-s-triazin
4) 2,4-Bis(4'-aminobenzaldi(2-ethylhexyl)malonat)-6-(4'-amino-2-ethylhexylbenzoat)-s-triazin
5) 2,4-Bis(4'-aminobenzaldiisobutylmalonat)-6-butoxy-s-triazin
6) 2,4-Bis(4'-aminobenzaldiisobutylmalonat)-6-(2-ethylhexylamino)-s-triazin
7) 2,4-Bis(4'-aminobenzaldiisobutylmalonat)-6-(5'-amino-2-ethylhexylsalicylat)-s-triazin
8) 2,4-Bis(4'-aminobenzaldiisobutylmalonat)-6-(4'-amino-2-ethylhexylcinnamat)-s-triazin
9) 2,4-Bis(4'-aminobenzaldiisobutylmalonat)-6-(4'-aminobenzylidencampher)-s-triazin.

3. Verfahren zur Herstellung der Verbindung der Formel (I) gemäß Anspruch 1 oder 2,
dadurch **gekennzeichnet,** daß
man eine Reaktion gemäß dem folgenden Reaktionsschema durchführt: 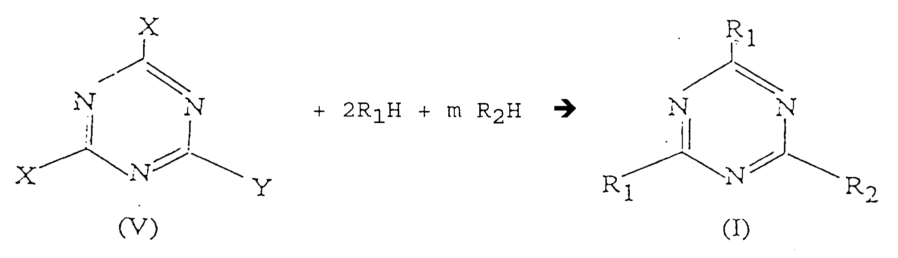 worin R₁ die in Anspruch 1 angegebene Formel (II) und R₂ die Formel (II), (III) oder (IV) des Anspruchs 1 aufweisen, X ein Halogenatom darstellt, Y ein Halogenatom, eine lineare oder verzweigte Alkoxy- oder Mono- oder Dialkylaminogruppe mit 1 bis 20 Kohlenstoffatomen darstellt, m gleich 1 ist, wenn R₂ die Formel (II), (III) oder (IV) hat, und m gleich 0 in den anderen Fällen ist,
wobei die Reaktion gegebenenfalls in Gegenwart eines Lösungsmittels wie Toluol, Xylol oder einer Mischung aus Aceton/Wasser bei einer Temperatur von 0 bis 250°C, vorzugsweise von 0 bis 150°C, durchgeführt wird.

4. Kosmetische Zusammensetzung,
dadurch **gekennzeichnet,** daß
sie eine wirksame Menge mindestens einer Verbindung der Formel (I) gemäß Anspruch 1 oder 2 in einer kosmetisch geeigneten Trägerzusammensetzung enthält, die mindestens eine Fettphase oder ein organisches Lösungsmittel umfaßt.

5. Kosmetische Zusammensetzung gemäß Anspruch 4,
dadurch **gekennzeichnet,** daß
sie mindestens eine Verbindung enthält, die aus den folgenden Verbindungen ausgewählt ist:
1) 2,4,6-Tris(4'-aminobenzaldiisobutylmalonat)-s-triazin
2) 2,4-Bis(4'-aminobenzaldi(2-ethylhexyl)malonat)-6-chlor-s-triazin
3) 2,4,6-Tris(4'-aminobenzaldi(2-ethylhexyl)malonat)-s-triazin
4) 2,4-Bis(4'-aminobenzaldi(2-ethylhexyl)malonat)-6-(4'-amino-2-ethylhexylbenzoat)-s-triazin
5) 2,4-Bis(4'-aminobenzaldiisobutylmalonat)-6-butoxy-s-triazin
6) 2,4-Bis(4'-aminobenzaldiisobutylmalonat)-6-(2-ethylhexylamino)-s-triazin
7) 2,4-Bis(4'-aminobenzaldiisobutylmalonat)-6-(5'-amino-2-ethylhexylsalicylat)-s-triazin
8) 2,4-Bis(4'-aminobenzaldiisobutylmalonat)-6-(4'-amino-2-ethylhexylcinnamat)-s-triazin
9) 2,4-Bis(4'-aminobenzaldiisobutylmalonat)-6-(4'-aminobenzylidencampher)-s-triazin.

6. Kosmetische Zusammensetzung gemäß Anspruch 4 oder 5,
dadurch **gekennzeichnet,** daß
sie in Form einer öligen oder oleoalkoholischen Lotion, Emulsion, bläschenförmigen Dispersion aus amphiphilen ionischen oder nicht-ionischen Lipiden, eines oleoalkoholischen oder alkoholischen Gels, eines Feststoffstäbchens oder eines Aerosols vorliegt.

7. Kosmetische Zusammensetzung gemäß Anspruch 6,
dadurch **gekennzeichnet,** daß
sie ausserdem kosmetische Hilfstoffe enthält, ausgewählt aus Verdickungsmitteln, Weichmachern, Feuchtigkeitsmitteln, oberflächenaktiven Mitteln, Konservierungsstoffen, Antischaummitteln, Parfüm-Produkten, Ölen, Wachsen, Lanolin, Niedrigmonoalkoholen und -polyolen, Treibmitteln, Färbe- bzw. Farbmitteln und Pigmenten.

8. Kosmetische Zusammensetzung zum Schutz der menschlichen Haut gemäß einem der Ansprüche 4 bis 7,
dadurch **gekennzeichnet,** daß
sie 0,1 bis 2 Gew.% Verbindung der Formel (I) enthält.

9. Kosmetische Sonnenschutzmittelzusammensetzung gemäß einem der Ansprüche 4 bis 7,
dadurch **gekennzeichnet,** daß
sie 0,3 bis 15 Gew.% Verbindung der Formel (I) enthält.

10. Kosmetische Sonnenschutzmittelzusammensetzung gemäß Anspruch 9,
dadurch **gekennzeichnet,** daß
sie ausserdem ein Mittel enthält, das die UV-B- und/oder UV-A-Strahlen filtert, welches verschieden von der Verbindung der Formel (I) ist.

11. Kosmetische Zusammensetzung gemäß Anspruch 4 oder 5 zur Aufbringung auf die Haare,
dadurch **gekennzeichnet,** daß
sie in Form eines Schampoo, einer Lotion, eines Gels oder einer Emulsion zum Spülen, einer Lotion oder eines Gels zum Frisieren oder zur Behandlung, einer Lotion oder eines Gels zum Bürsten oder zur Wellengebung, eines Haarlacks, einer Zusammensetzung für die Dauerwelle, für die Entfärbung oder Färbung vorliegt und 0,1 bis 2 Gew.% Verbindung der Formel (I) enthält.

12. Kosmetische Zusammensetzung gemäß Anspruch 4 oder 5 in Form einer kosmetischen Zusammensetzung, die gegenüber der Einwirkung von Licht stabilisiert ist,
dadurch **gekennzeichnet,** daß
sie durch eine Zusammensetzung für Haare, ein Schminkprodukt oder eine Zusammensetzung zur Pflege oder Behandlung der Haut dargestellt ist, welche 0,1 bis 2 Gew.% Verbindung der Formel (I) enthält.

13. Nicht-therapeutisches Verfahren zum Schutz der Haut und natürlicher oder sensibilisierter Haare vor ultravioletter Strahlung,
dadurch **gekennzeichnet,** daß
man auf die Haut oder die Haare eine wirksame Menge einer kosmetischen Zusammensetzung aufbringt, die mindestens eine Verbindung der Formel (I) gemäß Anspruch 1 oder 2 enthält.

## Patentansprüche (Patentansprüche für folgende(n) Vertragsstaat(en): ES)

1. Verfahren zur Herstellung der Verbindung der folgenden Formel: 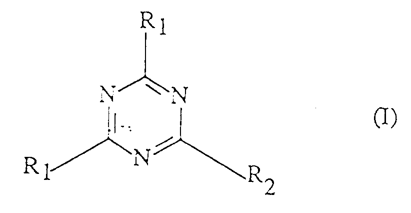 worin R₁ die Formel aufweist: 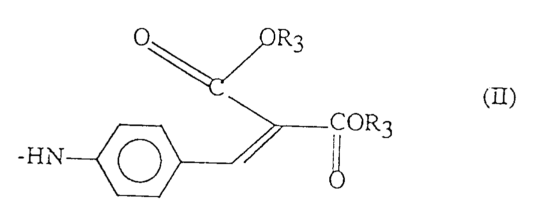 in welcher R₃ einen linearen oder verzweigten Alkylrest mit 1 bis 20 Kohlenstoffatomen darstellt,
und worin R₂ gleich R₁ ist oder ein Halogenatom, eine lineare oder verzweigte Alkoxy- oder Mono- oder Dialkylaminogruppe mit 1 bis 20 Kohlenstoffatomen oder eine Gruppe der folgenden Formel (III) oder der weiter unten angegebenen Formel (IV) darstellt: 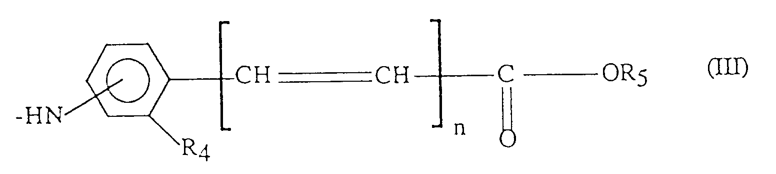 worin R₅ ein linearer oder verzweigter Alkylrest mit 1 bis 20 Kohlenstoffatomen ist und n = 0 oder 1, wobei gilt:
- wenn n gleich 0 ist, stellt R₄ ein Wasserstoffatom dar, wobei der Aminorest in Position 4 bezüglich der Carboxylgruppe vorliegt, oder stellt R₄ einen Hydroxylrest oder einen C₁₋₆-Alkoxyrest dar, wobei der Aminorest in Position 4 oder 5 bezüglich der Carboxylgruppe vorliegt;
- wenn n gleich 1 ist, stellt R₄ ein Wasserstoffatom dar, wobei der Aminorest in Position 4 bezüglich der ungesättigten Gruppierung vorliegt, 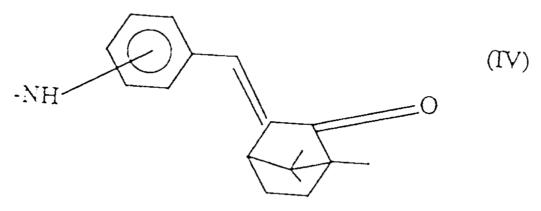 worin der Aminorest in Position 2, 3 oder 4 bezüglich der Methylidencamphergruppierung vorliegt,
mit der Maßgabe, daß gilt:
- wenn R₂ gleich R₁ ist, stellt R₃ einen Rest mit mindestens 4 Kohlenstoffatomen dar;
- wenn R₂ von R₁ verschieden ist und die Formel (III) aufweist, enthält R₅ mindestens 4 Kohlenstoffatome, wenn R₃ 1 bis 7 Kohlenstoffatome enthält;
- wenn R₂ verschieden von R₁ ist und nicht die Formel (III) aufweist, aber ein Alkoxy- oder Mono- oder Dialkylaminorest ist, weist R₂ mindestens 6 Kohlenstoffatome auf, wenn R₃ 1 bis 3 Kohlenstoffatome enthält,
dadurch **gekennzeichnet,** daß
eine Reaktion gemäß folgendem Reaktionsschema durchgeführt wird: 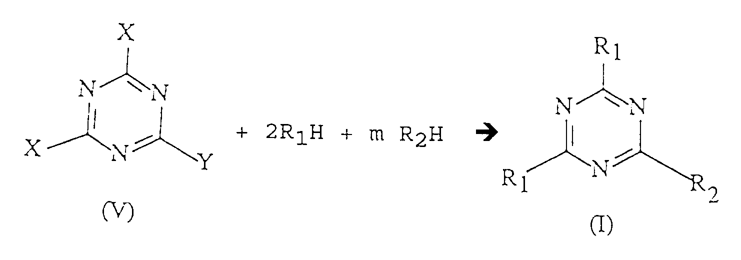 worin R₁ die in Anspruch 1 angegebene Formel (II) und R₂ die Formel (II), (III) oder (IV) des Anspruchs 1 aufweisen, X ein Halogenatom darstellt, Y ein Halogenatom, eine lineare oder verzweigte Alkoxy- oder Mono- oder Dialkylaminogruppe mit 1 bis 20 Kohlenstoffatomen darstellt, m gleich 1 ist, wenn R₂ die Formel (II), (III) oder (IV) hat, und m gleich 0 in den anderen Fällen ist,
wobei die Reaktion gegebenenfalls in Gegenwart eines Lösungsmittels wie Toluol, Xylol oder einer Mischung aus Aceton/Wasser bei einer Temperatur von 0 bis 250°C, vorzugsweise von 0 bis 150°C, durchgeführt wird.

2. Verfahren zur Herstellung der Verbindung der Formel (I) gemäß Anspruch 1,
dadurch **gekennzeichnet,** daß
man eine der folgenden Verbindungen herstellt:
1) 2,4,6-Tris(4'-aminobenzaldiisobutylmalonat)-s-triazin
2) 2,4-Bis(4'-aminobenzaldi(2-ethylhexyl)malonat)-6-chlor-s-triazin
3) 2,4,6-Tris(4'-aminobenzaldi(2-ethylhexyl)malonat)-s-triazin
4) 2,4-Bis(4'-aminobenzaldi(2-ethylhexyl)malonat)-6-(4'-amino-2-ethylhexylbenzoat)-s-triazin
5) 2,4-Bis(4'-aminobenzaldiisobutylmalonat)-6-butoxy-s-triazin
6) 2,4-Bis(4'-aminobenzaldiisobutylmalonat)-6-(2-ethylhexylamino)-s-triazin
7) 2,4-Bis(4'-aminobenzaldiisobutylmalonat)-6-(5'-amino-2-ethylhexylsalicylat)-s-triazin
8) 2,4-Bis(4'-aminobenzaldiisobutylmalonat)-6-(4'-amino-2-ethylhexylcinnamat)-s-triazin
9) 2,4-Bis(4'-aminobenzaldiisobutylmalonat)-6-(4'-aminobenzylidencampher)-s-triazin.

3. Kosmetische Zusammensetzung,
dadurch **gekennzeichnet,** daß
sie in einer kosmetisch geeigneten Trägerzusammensetzung, die mindestens eine Fettphase oder ein organisches Lösungsmittel umfaßt, eine wirksame Menge mindestens einer Verbindung der folgenden Formel (I) enthält: 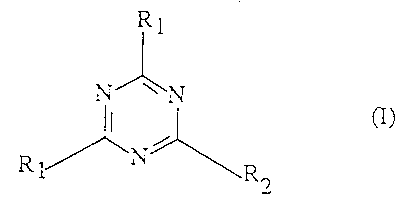 worin R₁ die Formel aufweist: 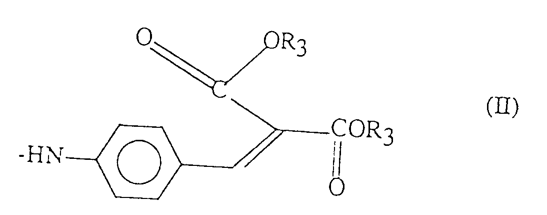 in welcher R₃ einen linearen oder verzweigten Alkylrest mit 1 bis 20 Kohlenstoffatomen darstellt,
und worin R₂ gleich R₁ ist oder ein Halogenatom, eine lineare oder verzweigte Alkoxy- oder Mono- oder Dialkylaminogruppe mit 1 bis 20 Kohlenstoffatomen oder eine Gruppe der folgenden Formel (III) oder der weiter unten angegebenen Formel (IV) darstellt: 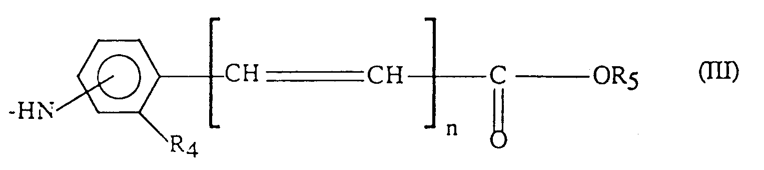 worin R₅ ein linearer oder verzweigter Alkylrest mit 1 bis 20 Kohlenstoffatomen ist und n gleich 0 oder 1, wobei gilt:
- wenn n gleich 0 ist, stellt R₄ ein Wasserstoffatom dar, wobei der Aminorest in Position 4 bezüglich der Carboxylgruppe vorliegt, oder stellt R₄ einen Hydroxylrest oder einen C₁₋₆-Alkoxyrest dar, wobei der Aminorest in Position 4 oder 5 bezüglich der Carboxylgruppe vorliegt;
- wenn n gleich 1 ist, stellt R₄ ein Wasserstoffatom dar, wobei der Aminorest in Position 4 bezüglich der ungesättigten Gruppierung vorliegt, 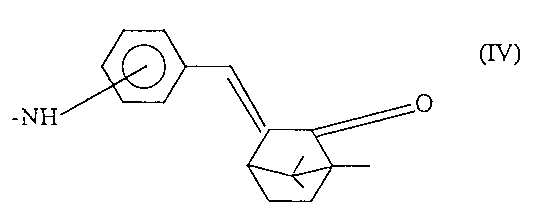 worin der Aminorest in Position 2, 3 oder 4 bezüglich der Methylidencamphergruppierung vorliegt,
mit der Maßgabe, daß gilt:
- wenn R₂ gleich R₁ ist, stellt R₃ einen Rest mit mindestens 4 Kohlenstoffatomen dar;
- wenn R₂ von R₁ verschieden ist und die Formel (III) aufweist, enthält R₅ mindestens 4 Kohlenstoffatome, wenn R₃ 1 bis 7 Kohlenstoffatome enthält;
- wenn R₂ verschieden von R₁ ist und nicht die Formel (III) aufweist, aber ein Alkoxy- oder Mono- oder Dialkylaminorest ist, weist R₂ mindestens 6 Kohlenstoffatome auf, wenn R₃ 1 bis 3 Kohlenstoffatome enthält,

4. Kosmetische Zusammensetzung gemäß Anspruch 3,
dadurch **gekennzeichnet,** daß
sie mindestens eine der Verbindungen enthält, die aus den folgenden Verbindungen ausgewählt sind:
1) 2,4,6-Tris(4'-aminobenzaldiisobutylmalonat)-s-triazin
2) 2,4-Bis(4'-aminobenzaldi(2-ethylhexyl)malonat)-6-chlor-s-triazin
3) 2,4,6-Tris(4'-aminobenzaldi(2-ethylhexyl)malonat)-s-triazin
4) 2,4-Bis(4'-aminobenzaldi(2-ethylhexyl)malonat)-6-(4'-amino-2-ethylhexylbenzoat)-s-triazin
5) 2,4-Bis(4'-aminobenzaldiisobutylmalonat)-6-butoxy-s-triazin
6) 2,4-Bis(4'-aminobenzaldiisobutylmalonat)-6-(2-ethylhexylamino)-s-triazin
7) 2,4-Bis(4'-aminobenzaldiisobutylmalonat)-6-(5'-amino-2-ethylhexylsalicylat)-s-triazin
8) 2,4-Bis(4'-aminobenzaldiisobutylmalonat)-6-(4'-amino-2-ethylhexylcinnamat)-s-triazin
9) 2,4-Bis(4'-aminobenzaldiisobutylmalonat)-6-(4'-aminobenzylidencampher)-s-triazin.

5. Kosmetische Zusammensetzung gemäß Anspruch 3 oder 4,
dadurch **gekennzeichnet,** daß
sie in Form einer öligen oder oleoalkoholischen Lotion, Emulsion, bläschenförmigen Dispersion aus amphiphilen ionischen oder nicht-ionischen Lipiden, eines oleoalkoholischen oder alkoholischen Gels, eines Feststoffstäbchens oder eines Aerosols vorliegt.

6. Kosmetische Zusammensetzung gemäß Anspruch 5,
dadurch **gekennzeichnet,** daß
sie ausserdem kosmetische Hilfstoffe enthält, ausgewählt aus Verdickungsmitteln, Weichmachern, Feuchtigkeitsmitteln, oberflächenaktiven Mitteln, Konservierungsstoffen, Antischaummitteln, Parfüm-Produkten, Ölen, Wachsen, Lanolin, Niedrigmonoalkoholen und -polyolen, Treibmitteln, Färbe- bzw. Farbmitteln und Pigmenten.

7. Kosmetische Zusammensetzung zum Schutz der menschlichen Haut gemäß einem der Ansprüche 3 bis 6,
dadurch **gekennzeichnet,** daß
sie 0,1 bis 2 Gew.% Verbindung der Formel (I) enthält.

8. Kosmetische Sonnenschutzmittelzusammensetzung gemäß einem der Ansprüche 3 bis 6,
dadurch **gekennzeichnet,** daß
sie 0,3 bis 15 Gew.% Verbindung der Formel (I) enthält.

9. Kosmetische Sonnenschutzmittelzusammensetzung gemäß Anspruch 8,
dadurch **gekennzeichnet,** daß
sie ausserdem ein Mittel enthält, das die UV-B- und/oder UV-A-Strahlen filtert, welches verschieden von der Verbindung der Formel (I) ist.

10. Kosmetische Zusammensetzung gemäß Anspruch 3 oder 4 zur Aufbringung auf die Haare,
dadurch **gekennzeichnet,** daß
sie in Form eines Schampoo, einer Lotion, eines Gels oder einer Emulsion zum Spülen, einer Lotion oder eines Gels zum Frisieren oder zur Behandlung, einer Lotion oder eines Gels zum Bürsten oder zur Wellengebung, eines Haarlacks, einer Zusammensetzung für die Dauerwelle, für die Entfärbung oder Färbung vorliegt, und 0,1 bis 2 Gew.% Verbindung der Formel (I) enthält.

11. Kosmetische Zusammensetzung gemäß Anspruch 3 oder 4 in Form einer kosmetischen Zusammensetzung, die gegenüber der Einwirkung von Licht stabilisiert ist,
dadurch **gekennzeichnet,** daß
sie durch eine Zusammensetzung für Haare, ein Schminkprodukt oder eine Zusammensetzung zur Pflege oder Behandlung der Haut dargestellt ist, welche 0,1 bis 2 Gew.% Verbindung der Formel (I) enthält.

12. Verfahren zur Herstellung einer kosmetischen Zusammensetzung,
dadurch **gekennzeichnet,** daß
man 0,1 bis 15 Gew.% einer Verbindung der folgenden Formel (I): 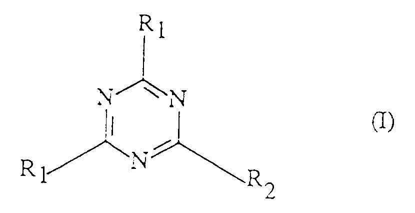 worin R₁ die Formel aufweist: 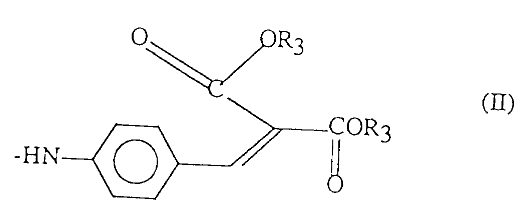 in welcher R₃ einen linearen oder verzweigten Alkylrest mit 1 bis 20 Kohlenstoffatomen darstellt,
und worin R₂ gleich R₁ ist oder ein Halogenatom, eine lineare oder verzweigte Alkoxy- oder Mono- oder Dialkylaminogruppe mit 1 bis 20 Kohlenstoffatomen oder eine Gruppe der folgenden Formel (III) oder der weiter unten angegebenen Formel (IV) darstellt: 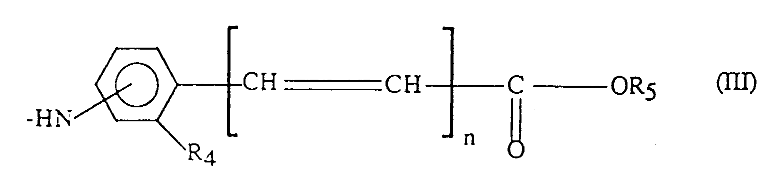 worin R₅ ein linearer oder verzweigter Alkylrest mit 1 bis 20 Kohlenstoffatomen ist und n = 0 oder 1, wobei gilt:
- wenn n gleich 0 ist, stellt R₄ ein Wasserstoffatom dar, wobei der Aminorest in Position 4 bezüglich der Carboxylgruppe vorliegt, oder stellt R₄ einen Hydroxylrest oder einen C₁₋₆-Alkoxyrest dar, wobei der Aminorest in Position 4 oder 5 bezüglich der Carboxylgruppe vorliegt;
- wenn n gleich 1 ist, stellt R₄ ein Wasserstoffatom dar, wobei der Aminorest in Position 4 bezüglich der ungesättigten Gruppierung vorliegt, 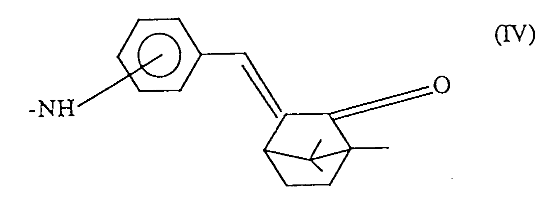 worin der Aminorest in Position 2, 3 oder 4 bezüglich de Methylidencamphergruppierung vorliegt,
mit der Maßgabe, daß gilt:
- wenn R₂ gleich R₁ ist, stellt R₃ einen Rest mit mindestens 4 Kohlenstoffatomen dar;
- wenn R₂ von R₁ verschieden ist und die Formel (III) aufweist, enthält R₅ mindestens 4 Kohlenstoffatome, wenn R₃ 1 bis 7 Kohlenstoffatome enthält;
- wenn R₂ verschieden von R₁ ist und nicht die Formel (III) aufweist, aber ein Alkoxy- oder Mono- oder Dialkylaminorest ist, weist R₂ mindestens 6 Kohlenstoffatome auf, wenn R₃ 1 bis 3 Kohlenstoffatome enthält,
in eine kosmetisch geeignete Trägerzusammensetzung einbringt, die mindestens eine Fettphase oder ein organisches Lösungsmittel umfaßt, ausgewählt aus Ölen, Wachsen, Fettkörpern, Niedrigmonoalkoholen und -polyolen und aus deren Mischungen.
